# EUROPEAN PATENT APPLICATION

(11) **EP 2 272 881 A2**
(43) Date of publication of application: **12.01.2011**
(21) Application number: 10182169.2
(22) Date of filing: 25.08.2001
(51) Int. Cl.: C08F 220/56, A61L 27/16, A61L 27/52, C08L 33/26

(54) **Polyacrylamide hydrogel for use in the treatment of arthritis**

(30) Priority: 25.08.2000 DK 200001262
(62) Divisional of application: 05009167.7
(71) Applicant: Contura A/S, 2860 Søborg (DK)
(72) Inventor: Lessel, Robert, 2605, Brøndby (DK); Schmidt, Richard, 2950, Vedbæk (DK)
(74) Representative: Plougmann & Vingtoft A/S

(57) **Abstract**

The invention concerns a hydrogel for use in the treatment of arthritis, said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. The preferred treatment is by injection of the hydrogel into the intra-articular cavity of a joint.

## Description

### FIELD OF INVENTION

The present invention relates to a novel polyacrylamide hydrogel cross-linked polyacrylamide. The hydrogel is obtainable by combining the acrylamide and methylene bis-acrylamide in a specific ratio so as to confer physical properties to the hydrogel. The present invention further relates to the use of the hydrogel for the preparation of an endoprosthesis for cosmetic surgery, reconstructive surgery as a soft-tissue filler endoprosthesis, an endoprosthesis for the treatment of incontinence, an endoprosthesis for the treatment of arthritis, for mammoplasty and for the treatment of reflux oesophagitis.

### BACKGROUND OF THE INVENTION

Natural and synthetic polymers such as collagen, soya, glycerol, silicone, polyvinylpyrolidone and hyaluronic acid have been utilised as endoprostheses. Materials used for endoprostheses generally try to imitate the natural soft tissue and are intended to be safe to the health of the patient.

Polyacrylamide gels have also been disclosed. US 5,798,096 relates to a biocompatible hydrogel containing 3.5 to 6.0% cross-linked polyacrylamide. However, US 5,798,096 teaches that concentrations below 3.5% make the hydrogel unstable.

GB 2114578 relates to a polyacrylamide gel for medical and biological purposes containing 3 to 28% polyacrylamide with the remainder of the mass of the gel comprised of a physiological solution.

US 5,658,329 relates to an implantable endoprosthesis comprising a shell filled with a polyacrylamide gel comprising 2 to 20% polyacrylamide by weight and a viscosity range of 15 to 75 Pas.

Formacryl^{®} polyacrylamide is a soft-tissue endoprosthesis consisting of 5% reticulated polyacrylamide polymer and 95% apyrogenic water commercialised as an injectable device for medical and dental use to correct congenital or acquired deficits such as wrinkles, lines and scars. It is to be implanted with a syringue in the hypodermis.

US 5,306,404 relates to a process for preparing polyacrylamide gel plates for electrophoresis.

WO 99/10021 relates to an injectable, biocompatible hydrogel comprising 0.5 to 10% polyacrylamide and an antibiotic or antiseptic. WO 99/10021 is directed to the solving the problem of sappuration and rejection of the gel in its use an endoprosthesis.

### SUMMARY OF THE INVENTION

The generally invention relates to the biostable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution The bio-stable hydrogel typically has a molecular weight between 0.01 x 10⁶ and 20 x 10⁶. The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. The hydrogel is useful as an endoprosthetic amount, said gel tailored to the defect it seeks to correct.

The present invention relates in one aspect to a biocompatible hydrogel comprising i) less than 3.5 % (wt/wt) polyacrylamide cross-linked with methylene bis-acrylamide and ii) pyrogen-free water. Specifically, it relates to a hydrogel comprising less than 3.5% by weight polyacrylamide obtainable by combining acrylamide and methylene bis-acrylamide, radical initiation, and washing with pyrogen-free water, said combining being in a molar ratio of 150:1 to 1000:1.

An object of the invention is to provide a hydrogel for use as an injectable or implantable endoprosthesis comprising i) less than 3.5% (wt/wt) polyacrylamide cross-linked with methylene bis-acrylamide and ii) pyrogen-free water or saline solution. The present invention further relates to a method for the preparation of a hydrogel comprising the steps of combining acrylamide and methylene bis-acrylamide, radical initiation, and washing with pyrogen-free water so as to give less than 3.5% by weight polyacrylamide.

A further object of the invention is an injectable or implantable endoprosthesis comprising i) less than 3.5% by weight polyacrylamide cross-linked with methylene bis-acrylamide and ii) pyrogen-free water or saline solution. The invention further relates to the use of a hydrogel comprising i) less than 3.5% by weight polyacrylamide cross-linked with methylene bis-acrylamide and ii) pyrogen-free water or saline solution for the preparation of an endoprosthesis for cosmetic surgery, reconstructive surgery and therapy. A method for the preparation of an injectable or implantable endoprosthesis comprising the steps of mixing acrylamide and methylene bis-acrylamide in a ratio so as to give less than 3.5% by weight polyacrylamide, radical initiation, and washing with pyrogen-free water or saline solution is disclosed herein.

Moreover, the invention relates to a method of treatment of a cosmetic or functional defect with an injectable or implantable biocompatible endoprosthesis comprising:
a) preparation of a polyacrylamide hydrogel, said polyacrylamide hydrogel comprising less than 3.5% by weight and said polyacrylamide being cross-linked using methylene bis-acrylamide,
b) injection or implantation a sufficient amount of said hydrogel into a region of the body affected by a cosmetic or functional defect..

Hydrogels with higher polyacrylamide content are also embodied in the present invention particularly in relation to certain medical indications. A further object of the invention relates to the use of a hydrogel comprising i) more than 9.5% polyacrylamide by weight, based on the total weight of the hydrogel, and ii) pyrogen-free water or saline solution for the preparation of an implantable endoprosthesis for mammoplastic reconstruction.

A still further object of the invention relates to use of a hydrogel comprising more than 9.5% polyacrylamide by weight, based on the total weight of the hydrogel, for the preparation of an implantable endoprosthesis for body contouring.

The treatment of reflux oesophagitis is addressed in the present invention. The use of a hydrogel comprising more than 6% polyacrylamide by weight, based on the total weight of the hydrogel, for the preparation of an implantable endoprosthesis for treating (reflux) oesophagitis is a further object of the invention as is a method for treating (reflux) oesophagitis comprising implanting or injecting a polyacrylamide hydrogel endoprosthesis wherein the hydrogel comprises more than 6% polyacrylamide by weight, based on the total weight of the hydrogel.

Mammoplasty is also addressed herein. The present invention further relates to a method of cosmetically altering a mammalian breast or of performing a partial or total mammoplastic reconstruction on a woman comprising implanting a polyacrylamide hydrogel ; wherein said hydrogel comprises more than 9.5% polyacrylamide by weight, based on the total weight of the hydrogel, and ii) pyrogen-free water or saline solution.

Cosmetic surgery on the body (excluding the face), referred to herein as body contouring is also addressed herein. The present invention is further directed to a method of cosmetically altering a mammalian body (body contouring) comprising implanting a polyacrylamide hydrogel endoprosthesis, wherein the hydrogel comprises more than 9.5% polyacrylamide by weight, based on the total weight of the hydrogel, and ii) pyrogen-free water or saline solution.

In another aspect, the invention relates to a hydrogel for use as a soft tissue filler endoprosthesis said hydrogel obtainable by combining acrylamide and methylene bis-acrylamide, radical initiation; and washing with pyrogen-free water or saline solution so as to give less than 3.5% by weight polyacrylamide, based on the total weight of the hydrogel.

In a further aspect, the invention relates to the use of a hydrogel comprising less than 3.5% by weight polyacrylamide, based on the total weight of the hydrogel, for the preparation of an endoprosthesis for soft tissue filling. Similarly, the invention relates to a method of filling soft tissue in a mammal comprising administering an endoprosthesis wherein the endoprosthesis comprises a hydrogel comprising less than 3.5% by weight polyacrylamide, based on the total weight of the hydrogel.

A further object of the invention is to provide a prosthetic device for soft tissue augmentation said device being injectable and comprising a polyacrylamide hydrogel said hydrogel being obtainable by combining acrylamide and methylene bis-acrylamide; radical initiation; and washing with pyrogen-free water or saline solution, so as to give less than 3.5% by weight polyacrylamide, based on the total weight of the hydrogel;

An important aspect of the invention relates to a bio-stable hydrogel for use in the in the treatment and prevention of incontinence and vesicouretal reflux, said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

A further aspect of the invention relates to the use of a bio-stable hydrogel comprising about 0.5 to 25% by weight polyacrylamide, based on the total weight of the hydrogel, for the preparation of an endoprosthesis for the treatment and prevention of incontinence and vesicouretal reflux and to a method of treating or preventing incontinence or vesicouretal reflux comprising administering a hydrogel to a mammal said hydrogel comprising 0.5 to 25% by weight polyacrylamide, based on the total weight of the hydrogel.

An important object of the invention is to provide a prosthetic device for increasing the resistance of conduits selected from the group consisting of the urethra; the rectum or colon; and the ureter for the treatment of urinary incontinence, anal incontinence, and vesicouretal reflux, respectively; wherein said device is injectable and comprising the biostable hydrogel of the invention.

One object of the invention is to provide a polyacrylamide hydrogel for use as a prosthetic device for supplementing, augmenting or replacing cartilage in the intra-articular cavity of a joint. The soft material has at least two advantageous features in that the material is firstly both biocompatible and biostable; and secondly, the material is mechanically resilient and does not bead, tear, shred or disintegrate readily upon mechanical stress. The material may be injected or implanted and manipulated so as to distribute the support provided by the material uniformly or according to the needs of the patient. The hydrogel also provides lubrication to the joint and the pre-existing cartilage.

A central object of the invention is to provide a hydrogel for use in the in the treatment or prevention of arthritis said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

A further aspect of the invention relates to the use of a hydrogel comprising about 0.5 to 25% by weight polyacrylamide, based on the total weight of the hydrogel, for the preparation of an endoprosthesis for alleviation or prevention of symptoms associated with arthritis.

Furthermore, providing a method of treating or preventing arthritis comprising administering a hydrogel to a mammal said hydrogel comprising 0.5 to 25% by weight polyacrylamide, based on the total weight of the hydrogel is further object of the invention.

Another aspect of the invention relates to a prosthetic device for the treatment of arthritis, wherein the device comprises a polyacrylamide hydrogel comprising 0.5 to 25% by weight polyacrylamide, based on the total weight of the hydrogel, said device administered to the intra-articular cavity of joint. Alternatively defined, the prosthetic device of the invention is for augmenting or replacing cartilage in the intra-articular cavity of a joint, said device comprises a polyacrylamide hydrogel comprising 0.5 to 25% by weight polyacrylamide, based on the total weight of the hydrogel.

### DETAILED DESCRIPTION OF THE INVENTION

### Hydrogels and Their Preparation

The success of plastic or reconstructive surgery depends to a great extent on the physical properties of the materials utilised. They must most certainly be biocompatible, stable and non-toxic but they must also have physical properties that mimic the bodily tissue they are replacing, as in reconstructive surgery, or mimic the bodily tissue in the proximity of the endoprosthesis, as in cosmetic surgery.

Materials such as collagen are resorbed into the body over short periods of time. Silicone and Soya have encountered serious safety issues. There is currently a need for a safe, stable, biocompatible material that possesses the physical properties to mimic soft tissue.

The present inventors have surprisingly found that a polyacrylamide hydrogel comprising less than 3.5% polyacrylamide, based on the total weight of the hydrogel, is an effective endoprosthesis with advantageous physical properties. Contrary to US 5,798,096, the polyacrylamide gel is stable. The hydrogel, as prepared by a process according to the present invention, is cross-linked with methylene bis-acrylamide to a degree such that the endoprosthesis prepared from said hydrogel possesses advantageous physical characteristics. The hydrogel according to the present invention is a new chemical entity as indicated by its novel and advantageous physical characteristics. These secondary characteristics are indicative that the degree of cross-linking in the hydrogel of the present invention differs greatly from polyacrylamide hydrogels prepared by disclosed processes. This degree of cross-linking is a critical contributor to its physical properties.

The present investigators have provided a bio-stable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution. The bio-stable hydrogel typically has a molecular weight between 0.01 x 10⁶ and 20 x 10⁶. The polymer is resistant to biological , degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

A primary object of the invention is to provide a hydrogel comprising less than 3.5% polyacrylamide by weight, based on the total weight of the hydrogel, said hydrogel obtainable by combining acrylamide and methylene bis-acrylamide, radical initiation, and washing with pyrogen-free water or saline solution; said hydrogel being biocompatible, and said combining being in a molar ratio of 150:1 to 1000:1.

Alternatively defined, one aspect of the invention relates to hydrogel comprising i) less than 3.5 % polyacrylamide by weight, based on the total weight of the hydrogel, cross-linked with methylene bis-acrylamide and ii) at least 95% pyrogen-free water or saline solution.

A further aspect of the invention relates to a hydrogel for use as an injectable or implantable endoprosthesis said hydrogel comprising i) less than 3.5% polyacrylamide by weight, based on the total weight of the hydrogel, cross-linked with methylene bis-acrylamide and ii) at least 95% pyrogen-free water or saline solution.

In all embodiments wherein the hydrogel comprises less than 3.5% polyacrylamide by weight, based on the total weight of the hydrogel, the hydrogel typically further comprises at least 95% pyrogen-free water or saline solution.

The hydrogel comprises less than 3.5% polyacrylamide preferably comprising at least 0.5%, such as at least 1 %, preferably at least 1.5% polyacrylamide, such as at least 1.6% polyacrylamide by weight, based on the total weight of the hydrogel.

The hydrogel comprising less than 3.5% polyacrylamide are chemically stable and biostable but may be very fluid such that they may be characterised in that it has a complex viscosity not less than 2 Pas, such as not less than 3, 4 or 5 Pas. In a suitable embodiment, the hydrogel comprising less than 3.5% polyacrylamide is characterised in that it has complex viscosity from about 2 to 90, such as 5 to 80 Pas, preferably from about 6 to 76, such as from about 6 to 60, 6 to 40, 6 to 20, such as 6 to 15 Pas.

The hydrogel comprising less than 3.5% polyacrylamide has elastic properties in that the hydrogel may be characterised in that it has elasticity module of not less than 10 Pa, such as not less than 20, 25, 30, 31, 32, 33, 34 or 35 Pa, such as not less than 38 Pa. Typically, the hydrogel has an elasticity module from about 10 to 700 Pa, such as about 35 to 480 Pa.

These rheological features are in due in part to the degree of cross-linking and to the degree of swelling of the hydrogel. The hydrogel comprising less than 3.5% polyacrylamide may be characterised in that the cross-linked polyacrylamide is to such as degree so as to have an efficient cross-linking density of about 0.2 to 0.5%, preferably about 0.25 to 0.4%.

The cross-linking density is in turn due in part to the molar ratio between the acrylamide and methylene-bis-acrylamide. Typically said ratio is in the range 175:1 to 800:1, such as from 225:1 to 600:1, preferably from 250:1 to 550:1, most preferably from 250:1 to 500:1. The absolute and relative amount of the redox agent (TEMED) and the initiator also influence the degree of cross-linking. As can be seen from Tables 1, 2, 3, 4, the present investigators have adjusted these parameters to influence the rheological properties of the hydrogel.

The biocompatible hydrogels of the invention comprising less than 3.5% polyacrylamide may be suitably characterised, at least in part, by one or more of the following features: i) a cross-linking density of 0.2% to 0.5 %; ii) an elasticity modulus (G') of 10 to 700 Pa; iii) a complex viscosity of 2 to 90 Pa s; iv) a dry matter content of less than 3.5% such as less than 3.4, such as less than 3.3, such as less than 3.2, such as less than 3.1, such as less than 3.0, such as less than 2.9, such as less than 2.8, such as less than 2.7, such as less than 2.6 Pa s; v) a refractive index between 1.33 and 1.34.

As stated, in all aspects of the invention wherein the hydrogel comprises less than 3.5% polyacrylamide by weight, based on the total weight of the hydrogel, the hydrogel further comprising at least 95% pyrogen-free water or saline solution. In all embodiments wherein the hydrogel further comprises saline solution, the hydrogel preferably comprises less than 3 % polyacrylamide by weight, based on the total weight of the hydrogel.

The hydrogel of the invention is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

The hydrogel comprises pyrogen-free water or saline solution. The combining of the reagents and the casting of the gel may therefore be done in pyrogen-free water or saline solution. The use of saline solution will clearly increase the total solid weight content of the hydrogel but does not significantly influence the polyacrylamide content during the polymerisation reaction.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is an about 0.8- 1% aqueous sodium chloride solution, such as a 0.8, 0.9 or 1 % aqueous sodium chloride solution.

As stated, pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm. In the method of the invention, the washing step comprises swelling the product for 50 to 250 hours, more typically for 70 to 200 hours.

The hydrogel comprising less than 3.5% polyacrylamide was surprisingly found to be stable at very low solid weight content, contrary to the teachings of US 5,798,096.

Polyacrylamide hydrogels with a solid-weight content of 0.5% have been prepared by the present investigators. Preferred embodiments of the hydrogel of the invention comprise at least 0.5%, such as at least 1 %, preferably at least 1.5% polyacrylamide, such as at least 1.6% polyacrylamide by weight, based on the total weight of the hydrogel.

The hydrogel is suitably a composite of cross-linked polyacrylamide chains and pyrogen-free water. Water contained in the hydrogel as part of a composite is loosely bound with the polymer chains. When present in a body, some of the water molecules move into the tissue by osmosis resulting in a smoothing out of the affected skin surface. In the embodiment wherein the hydrogel comprises saline solution, the iso-osmolarity of the saline solution with interstitial fluid minimises immune responses.

As mentioned, the physical properties of the hydrogel are influenced in part by the degree of cross-linking. The degree of cross-linking may be controlled in part by the molar ratio of cross-linking agent, methylene bis-acrylamide, to acrylamide.

Therefore, another object of the invention is a method for the preparation of a hydrogel comprising the steps of combining acrylamide and methylene bis-acrylamide, radical initiation, and washing with pyrogen-free water or saline solution so as to give less than 3.5% by weight polyacrylamide, based on the total weight of the polyacrylamide. The method is preferably such that the hydrogel comprises at least 0.5%, such as at least 1 %, preferably at least 1.5% polyacrylamide, such as at least 1.6% polyacrylamide by weight, based on the total weight of the hydrogel.

In a especially preferred embodiment of the invention, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution.

The combining of acrylamide and methylene bis-acrylamide in preferably done in a molar ratio between acrylamide and methylene bis-acrylamide is from 175:1 to 800:1, such as from 225:1 to 600:1, preferably from 250:1 to 550:1, most preferably from 250:1 to 500:1.

An illustrative preparation of the hydrogel according to the present invention is described in Example 1. The hydrogel having the desired physical properties has been obtained by combining acrylamide and methylene bis-acrylamide in a ratio of about 250:1, such as 252:1, 254:1, 256:1, 258:1 and 260:1, as well as by combining acrylamide and methylene bis-acrylamide in a ratio of about 500:1, such as 498:1, 496:1 494:1 492:1, or 490:1. The hydrogel according to the present invention preferably has a complex viscosity from about 2 to 90, such as 5 to 80 Pas, typically from about 6 to 76, such as from about 6 to 60, 6 to 40, 6 to 20, such as 6 to 15 Pas. In a suitable embodiment, the washing step comprises swelling the product of the radical initiation step until the complex viscosity is from about 6 to 100 Pas.

In a suitable embodiment of the invention, the hydrogel has a degree of cross-linking such that it has complex viscosity of not less than 2 Pas, such as not less than 3, 4 or 5 Pas, such as not less than 5.5 Pas, such as not less than 6 Pas, preferably not less than 6.2 Pas.

The elasticity module is an alternative physical characteristic of the hydrogel indicative, in part, of the degree of cross-linking of the hydrogel according to the present invention. Typically, the degree of cross-linking is such that the hydrogel has an elasticity module of not less than 10 Pa, such as not less than 25, 30, 31, 32, 33, 34 or 35 Pa, such as not less than 38 Pa. The gel may be characterised in that it has elasticity module from about 10 to 700 Pa, such as about 35 to 480 Pa.

As stated, in a most preferred embodiment, the hydrogel is obtainable by combining acrylamide and methylene bis-acrylamide in amounts so as to give 1.6 to 3.5% by weight polyacrylamide and in a molar ratio of 150:1 to 1000:1, radical initiation, and washing with pyrogen-free water or saline solution. The radical initiation step yields a hydrogel still comprising toxic reagents and not yet possessing the beneficial physical properties of the hydrogel of the present invention. The washing step comprises swelling the hydrogel produced from the radical initiation step until the complex viscosity is from about 2 to 90 Pas.

Alternatively measured, the washing step may comprise swelling the product of the radical initiation step until the elasticity module is elasticity module from about 10 to 700 Pa, such as from about 35 to 480 Pa.

By nature, a low degree of cross-linking typically results in a higher swelling rate consequently lowering the dry matter content (percentage acrylamide), as well as lowering the elasticity module and viscosity. Thus, in addition to the degree of cross-linking, the time the hydrogel is exposed to the washing step influences to a degree the physical properties of the gel.

Typically, the washing step comprises swelling the product for about 80 to 100 hours, such as 90-95 hours. This usually results in an increase in weight of the hydrogel of about 75 to 150%, usually around 100% increase.

Furthermore, the amounts of the free-radical initiator in the radical initiation step and the amount of co-initiator influences the chain length and thus the physical properties of the hydrogel. In a typical preparation of the hydrogel, N-,N-,N-,N-tetramethyl ethylene diamine (TMED) is used as the co-initiator and ammoniumpersulfate (APS) is used as the free-radical initiator (redox-system). Adequate amounts of initiator and co-initiator are required to obtain the hydrogel according to the invention. As an illustration, an insufficient amount of these reagents will result in shorter chain lengths and consequently influence the degree of cross-linking and hence the physical properties of the hydrogel. Other reaction conditions, such as temperature also influence chain length.

As stated, the degree of cross-linking influences the physical properties of the hydrogel. The degree of cross-linking of the hydrogel of the present invention may be indirectly measured, as described above, by the elasticity module and/or by the complex viscosity. An alternative measure of the degree of cross-linking of the hydrogel is its efficient cross-linking density. The hydrogel of the present invention preferably comprises 1.6 to 3.5% (wt/wt) polyacrylamide cross-linked with methylene bis-acrylamide and ii) pyrogen-free water or saline solution, preferably such that the degree of cross-linking as measured by its efficient cross-linking density is such that the efficient cross-linking density is about 0.2 to 0.5%, preferably about 0.25 to 0.4%.

In a suitable embodiment of the present invention, the hydrogel may comprise 1.6 to 3.25% (wt/wt) polyacrylamide, such as 1.8 to 3.1, 2.0 to 3.0, 2.0 to 2.9, preferably 2.0 to 2.8 (wt/wt) polyacrylamide.

### Medical Use of Polyacrylamide Hydrogels

The hydrogels of the invention are intended for use as endoprosthetic devices. The endoprosthetic devices of the invention and methods of treatment described herein may use the hydrogel in any of the embodiments described supra.

The endoprosthetic device may be administered to the body of an individual by means of injection, such as through a syringe or catheter or by means of surgical implantation. In the embodiment wherein the hydrogel is for use as an implantable endoprosthesis, the hydrogel may optionally serve as filler material in an envelope, the whole of which is implanted into the body. Thus the hydrogel or the endoprosthesis may comprise a silicone-based envelope housing the hydrogel.

As stated, an object of the invention is a method of treatment of a cosmetic or functional defect with an injectable biocompatible endoprosthesis comprising:
a) preparation of a 1.6 to 3.5% by weight polyacrylamide hydrogel, said polyacrylamide cross-linked using methylene bis-acrylamide,
b) injection a sufficient amount of said hydrogel into a region of the body affected by a cosmetic or functional defect. The hydrogel injected into an affected area is obtainable by combining acrylamide and methylene bis-acryalmide in amounts so as to give 1.6 to 3.5% by weight polyacrylamide and in a molar ratio of 150:1 to 1000:1, radical initiation, and washing with pyrogen-free water or saline solution. The ratio of acrylamide to methylene bis-acrylamide is preferably such that the complex viscosity is from about 2 to 90, such as 5 to 80 Pas, preferably from about 6 to 76 Pas or such that the elasticity module is from about 10 to 700 Pa, such as about 35 to 480 Pa. The washing step may be done to the extent such that the complex viscosity is from about 6 to 80 Pas or such that the elasticity module is from 10 to 700 Pa, such as about 35 to 480 Pa.

The endoprosthetic device may comprise any of embodiments of the hydrogels discussed herein and may be either implantable or injectable Thus an important aspect of the present invention is the use of a hydrogel comprising i) less than 3.5% by weight polyacrylamide cross-linked with methylene bis-acrylamide and ii) at least 95% pyrogen-free water or saline solution for the preparation of an endoprosthesis for cosmetic surgery, reconstructive surgery and therapy. In particularly interesting embodiments of invention, the endoprosthesis is injectable.

A further object of the invention is a hydrogel as described *supra* for use in the preparation of an endoprosthesis. Specifically, an object of the invention is the use of a hydrogel comprising i) 1.6 to 3.5% by weight polyacrylamide cross-linked using methylene bis-acrylamide and ii) pyrogen-free water or saline solution for the preparation of an endoprosthesis for cosmetic surgery, reconstructive surgery and therapy. The endoprosthesis according to the present invention may be implantable or injectable. The cosmetic and reconstructive surgery may be for facial correction such as for altering the form or size of lips, to treat wrinkles or facial asymmetries, or altering the shape of a nose as well as a variety of facial corrections known to the person skilled in the art.

Furthermore, cosmetic and reconstructive surgery may be to correct defects caused by trauma or due to diseases such as, for instance, hemiphlegia.

A hydrogel as described *supra* may be used for the preparation of an endoprosthesis. Thus, a further object of the present invention is an injectable endoprosthesis comprising i) 1.6 to 3.5% by weight polyacrylamide cross-linked with methylene bis-acrylamide and ii) pyrogen-free water or saline solution. An injectable endoprosthesis according to the present invention preferably has a viscosity complex from about 2 to 90, such as 5 to 80 Pas, preferably from about 6 to 76, such as from about 6 to 60, 6 to 40, 6 to 20, such as 6 to 15 Pas.

Wherein the endoprosthesis is injected, the hydrogel is generally somewhat fluid in nature.

Many disorders are related to a loss of effective activity of the tissue at a functional interface between two organs. For instance, urinary incontinence is related to insufficient sphincter between the urinary bladder and the urethra. By injecting or implanting an endoprosthesis prepared from the hydrogel according the present invention into the proximal submucosa of the urethra, thereby narrowing the urethra, the disorder may be significantly controlled. Similarly, reflux oesophagitis is related to insufficient resistance between the oesophagus and stomach. By injecting or implanting an endoprosthesis prepared from the hydrogel according the present invention along the sphincter between the oesophagus and stomach, the contact between the contents of the stomach and the oesophagus may be reduced. Thus, in suitable embodiments, the hydrogel is used for the preparation of an endoprosthesis treat urinary incontinence or reflux oesophagitis. The endoprosthesis may, in general, be used to treat disorders related to insufficient resistance at a functional interface between two organs or between segments of one organ.

The solid-weight content of weight percentage acrylamide, as measured after the washing step, as well as the degree of cross-linking is adapted according to the use of the endoprosthesis prepared from the hydrogel. In preferred embodiments, the endoprosthesis is preferably prepared from a hydrogel comprising 1.6 to 3.25% (wt/wt) polyacrylamide, such as 1.8 to 3.1, 2.0 to 3.0, 2.0 to 2.9, preferably 2.0 to 2.8 (wt/wt) polyacrylamide. Alternatively defined, the degree of cross-linking of the hydrogel for use as an endoprosthesis may preferably be such that the complex viscosity of the hydrogel is from about 2.0 to 15 Pas such as from about 5.5 to 15 Pas, such as from 6 to 12 Pas. Alternatively measured, for facial corrections the degree of cross-linking of the hydrogel are preferably such that the elasticity module is from about 10 to 100 Pa, such as about 35 to 75, particularly 35 to 60 Pa, such as 35 to 50 Pa.

The endoprosthesis may be to correct a defect which is the result of trauma such as tumours or physical injury as well as congenital defects.

In a suitable embodiment of the present invention, the hydrogel as described *supra* is used as a filling material to fill or partially fill a silicone-based reservoir or shell for the preparation of an implantable endoprosthesis. Thus, a further object of the present invention is an implantable endoprosthesis comprising i) a hydrogel comprising 1.6 to 3.5% (wt/wt) polyacrylamide cross-linked with methylene bis-acrylamide and pyrogen-free water or saline solution ii) a silicone-based shell adapted for containing said hydrogel. The hydrogel used to fill or partially fill the silicone-based shell may be a hydrogel obtainable by combining acrylamide and methylene bis-acryalmide in amounts so as to give 1.6 to 3.5% by weight polyacrylamide and in a molar ratio of 150:1 to 1000:1, radical initiation, and washing with pyrogen-free water or saline solution. In embodiments wherein the endoprosthesis is implantable, the preparation of said endoprosthesis from a hydrogel optionally further comprises the step of inserting said hydrogel into a silicone-based shell.

After the hydrogel has been placed into the silicone-based shell, it can preferably be sealed by chemical, mechanical or thermal means or by laser or light. Typically, the outer surface of the shell is chemically or physically modified such that it is biocompatible and have a friction coefficient so as to minimise movement or slippage from the position it was placed.

In an alternative embodiment of the invention, the endoprosthesis may comprise a medicament for use in therapy.

### Polyacrylamide Hydrogel as a Soft-Tissue Filler Endoprosthesis

The term "facial" in this aspect of the invention intended mean of relation to all areas of the face, such as but not exclusively, the cheeks, the jaw, the neck, the forehead, under the eyes, the head area, and the nose.

The term "body contouring" is intended to mean cosmetic or reconstructive surgery wherein soft tissue is augmented in order to correct a cosmetic or non-cosmetic defect in the soft tissue in the body, excluding the face, lips, breasts and penis. In this aspect of the invention, the endoprosthesis is directed to facial corrections, lip augmentation, and body contouring.

In this aspect of the invention, the term hydrogel relates to the polyacrylamide polymer of the invention comprising less than 3.5% polyacrylamide and at least 95% pyrogen-free water or saline solution whereas the term endoprosthesis relates to the hydrogel present in the body.

The polyacrylamide hydrogel is obtainable by the polymerisation of the monomers acrylamide and N,N'-methylene-bis-acrylamide under radical initiation, followed by washing of the polymer with pyrogen-free water or saline solution. The washing of the polymer results in a swelling of the gel, due to absorption of the pyrogen-free water or saline solution by the polymer. The swelling of the hydrogel influences the solid weight content of the gel, i.e. the amount of polymeric material, polyacrylamide. The solid weight content of the hydrogel influences, at least in part, the physical (rheological) properties of the hydrogel and thus the ability to mimic human tissue when used as an endoprosthesis.

The present investigators have prepared a hydrogel having the desired rheological properties to act as a soft tissue filler endoprosthesis which is completely atoxic, stable, and non-resorbable. The present investigators have developed the hydrogel to be particularly amenable for use as an endoprosthesis for facial cosmetic or reconstructive surgery, for body contouring and for lip augmentation or reconstruction. The hydrogel in this aspect of the invention is not directed for use as an endoprosthesis for breast or penis augmentation.

A first aspect of in this aspect of the invention relates to a hydrogel for use as a soft tissue filler endoprosthesis said hydrogel obtainable by combining acrylamide and methylene bis-acrylamide in amounts so as to give less than 3.5% by weight polyacrylamide, based on the total weight of the hydrogel; radical initiation; and washing with pyrogen-free water or saline solution. Typically, the hydrogel is obtained by said combining acrylamide and methylene bis-acrylamide in a molar ratio of 150:1 to 1000:1. The hydrogel obtained in this manner has a structural formula as shown in Figure 1, is sterile, has transparent or colourless appearance and has a pH in the range of 6.5 to 9.0, typically 7.0 to 9.0. Furthermore the hydrogel of the invention is stable to oxygen, high pressure, high and low temperatures, enzymes and bacteria.

This aspect of the invention thus relates to the use of a hydrogel comprising less than 3.5% by weight polyacrylamide, based on the total weight of the hydrogel, for the preparation of an endoprosthesis for soft tissue filling. Given the hydrogel in this aspect of the invention is directed for use as an endoprosthesis, it must be stable. Furthermore, given the hydrogel of the invention is directed for use as an endoprosthesis for selected parts of the human anatomy, the hydrogel typically comprises at least 0.5 % by weight polyacrylamide, based on the total weight of the hydrogel, preferably at least 1.0 % by weight polyacrylamide, more preferable at least 1.5 % by weight polyacrylamide, such as at least 1.6 % by weight polyacrylamide, based on the total weight of the hydrogel. Typically, the hydrogel of the present invention may have a solid weight content of 1.5, 1.6, 1.7 1.8, 1.9, 2.0, 2.1, 2.2, 2.3, 2.4, 2.5, 2.6, 2.7, 2.8, 2.9, 3.0, 3.1, 3.2, 3.3, 3.4, or 3.5% polyacrylamide, based on the total weight of the hydrogel.

In a preferred embodiment of this aspect of the invention, the hydrogel comprises about 1.9 to 2.9 % by weight polyacrylamide, based on the total weight of the hydrogel. The hydrogel typically further comprises at least 95 % by weight pyrogen-free water or saline solution, preferably pyrogen-free water. In a preferred embodiment, the hydrogel comprises at least 96 % by weight pyrogen-free water or saline solution, preferably pyrogen-free water, more preferably at least 97 % by weight pyrogen-free water or saline solution, preferably pyrogen-free water, such as 95 %, 95.5 %, 96 %, 96.5 %, 97 %, or 97.5 % by weight pyrogen-free water or saline solution, preferably pyrogen-free water.

A suitable saline solution has an osmolarity similar to that of interstitial fluid. Suitable saline solutions include but are not limited to the group selected from 0.25- 1% aqueous sodium chloride, a Ringer-Lockart solution, an Earle solution, a Hanks solution, an Eagle medium, a 0.25 - 1 % glucose solution, a potassium chloride solution, and a calcium chloride solution.. In a preferred embodiment, the saline solution is an about 0.8- 1 % aqueous sodium chloride solution, such as a 0.8, 0.9 or 1 % aqueous sodium chloride solution.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, particularly preferably below 20 ppm, typically below 10 ppm, most preferably less than 5 ppm.

The solid weight content of the hydrogel of the present invention is essentially completely contributed by the polyacrylamide and N,N'-methylene-bis-acrylamide with residual contribution by the initiator. The hydrogel is substantially free of any other polymeric content.

As stated, the hydrogel of the invention is biocompatible, atoxic, non-allergenic, non-resorbable, chemically inert and stable to oxygen, high pressure, high and low temperatures, enzymes and bacteria. In the event that the hydrogel is exposed to excessive amounts of UV light, the physical features of the hydrogel are altered and is converted into a glue-like substance. Advantageously, this substance is also non-toxic.

Upon administration of the hydrogel, a thin layer of connective tissue surrounds the endoprosthesis, enabling the endoprosthesis to become a stable part of the connective tissue. Due to the bio-stability of the hydrogel and the thin layer of connective tissue, the endoprosthesis may be easily removed from the patient when placed in the subcutaneous area. This advantage is at least in part due to the stability of the hydrogel which in turn is at least in part due to the washing process.

Several factors affect the rheological properties of the hydrogel, such as the relative amount of monomer used, the relative amount of initiator, the temperature, the time of polymerisation, and other parameters of the polymerisation process, as well as the washing process. Thus, the polymerisation process may provide a hydrogel with an array of viscosities having a solid weight content of less than 3.5%. The invention is directed to a hydrogel as a soft-tissue filler endoprosthesis and thus the hydrogel preferably has a viscosity tailored to the soft-tissue it is intended to mimic. The hydrogel of the invention is typically for use as an injectable endoprosthesis for cosmetic or reconstructive surgery of the face, cosmetic or reconstructive surgery of the body (body contouring), and augmentation or reconstructive surgery of the lips.

The hydrogel of this aspect of the invention may be injectable or implantable into the subcutaneous layer of the epidermis, preferably the hydrogel is injectable.

In one embodiment of this aspect of the invention, the endoprosthesis is for use in facial cosmetic or reconstructive surgery and the hydrogel has a complex viscosity of about 2 to 100 Pas, preferably about 5 to 90 Pas, such as about 5 to 60 Pas, such as about 10 to 60 Pas. Most preferably, the endoprosthesis for use in facial cosmetic or reconstructive surgery was place by means of injection of the hydrogel.

Depending on the condition and region of the epidermis of the face where the soft-tissue filler is required (e.g. chin as opposed to cheek), the viscosity of the hydrogel may vary. Accordingly, the hydrogel may be for use in facial cosmetic or reconstructive surgery and have a complex viscosity of about 2 to 20 Pas, preferably about 2 to 18 Pas, such as about 2 to 15 Pas or 2 to 10 Pas, more preferably 2 to 7 Pas, most preferably 3 to 5 Pas.

In typical embodiments, the endoprosthesis may be for uses in correction of facial contour deformities due to ageing, acne, trauma, surgery, infection or congenital deformities. The facial features typically in need of correction are, for instance, the cheekbones, nasolabial folds, glabellar frowns, depressed contours of the mouth, the chin, the size or shape the lips, as well as other soft tissue deficiencies of the face. The hydrogel restores the skin contours correcting soft tissue contour deformities of the face such as wrinkles and folds. The hydrogel may serve for the preparation of an injectable hydrogel for lip enhancement or correct an array of aesthetic defects caused by congenital, traumatic or ageing alterations.

As stated, this aspect of the invention relates to the use of the hydrogel for the preparation of an endoprosthesis for the filling of soft tissue filling selected from the soft tissue of the face and lips and soft tissue of the body. The hydrogel of the invention directed for use in the cosmetic or reconstructive surgery of the body (body contouring), preferably has a complex viscosity of about 5 to 50 Pas, preferably about 7 to 40 Pas, most preferably about 7 to 30 Pas.

In the embodiment wherein the hydrogel is used in the preparation of an endoprosthesis for lip augmentation or lip reconstruction the hydrogel preferably has a complex viscosity of about 2 to 10 Pas, more preferably 2 to 7 Pas, most preferably 3 to 5 Pas.

A further object of this aspect of the invention is to provide a prosthetic device for soft tissue augmentation said device being injectable and comprising a polyacrylamide hydrogel
said hydrogel being obtainable by combining acrylamide and methylene bis-acrylamide in amounts so as to give less than 3.5% by weight polyacrylamide, based on the total weight of the hydrogel; radical initiation; and washing with pyrogen-free water or saline solution.

The prosthetic device of this aspect of the invention preferably comprises a hydrogel comprising at least 0.5 % by weight polyacrylamide, based on the total weight of the hydrogel, preferably at least 1 % by weight polyacrylamide, more preferable at least 1.5 % by weight polyacrylamide, such as at least 1.6 % by weight polyacrylamide, based on the total weight of the hydrogel. Typically, the hydrogel comprises about 1.9 to 2.9 % by weight polyacrylamide, based on the total weight of the hydrogel. The prosthetic device comprises the hydrogel which typically comprises at least 95% by weight pyrogen-free water or saline solution, preferably pyrogen-free water.

A further aspect of the invention relates to a method of filling soft tissue comprising administering an endoprosthesis wherein the endoprosthesis comprises a hydrogel comprising less than 3.5% by weight polyacrylamide, based on the total weight of the hydrogel. The hydrogel may be as described *supra.*

In an alternative embodiment of the invention, the prosthetic device comprises cells, such as stem cells. Polyacrylamide provides an excellent template and matrix for cell growth. Although the hydrogel of the invention allows, in itself, for a thin layer of connective tissue from the body of the patient to surround the device, the use of cells in combination with the hydrogel of the invention for the preparation of the device allows for cellular engraftment to the surrounding tissue.

The method of this aspect of the invention typically comprises administering the hydrogel of the invention by injecting the hydrogel, into the subcutaneous layer of the skin in the embodiments wherein the endoprosthesis is for facial cosmetic or reconstructive surgery or body contouring. In the embodiment wherein the endoprosthesis is for lip augmentation or lip reconstruction, the injecting is above the muscle tissue of the lip.

A further object of the invention is the use of a hydrogel comprising i) more than 9.5% polyacrylamide by weight, based on the total weight of the hydrogel, and ii) pyrogen-free water or saline solution for the preparation of an implantable endoprosthesis for body contouring. This method of cosmetically altering a mammalian body (body contouring) comprising implanting a polyacrylamide hydrogel endoprosthesis, wherein the hydrogel comprises more than 9.5% polyacrylamide by weight, based on the total weight of the hydrogel, and ii) pyrogen-free water or saline solution may be performed by injection or implantation of the hydrogel into the soft-tissue of the patient.

As stated, an alternative embodiment of this aspect of the invention comprises administering the hydrogel of the invention in combination with cells, such as stem cells to allow for cellular engraftment of the prosthetic device.

The method of this aspect of the invention may comprise of more than one injection so as to cover the desired area or to achieve the desired affect. The gel to be injected is typically stored in a syringe suitable for injecting the required amount for a single session treatment. Depending on the afflicted area, the amount of gel and thus volume of the syringe may vary, such as a syringe with a volume of 0.25-25 mL, such as a syringe selected from 0.5 mL, 0.7 mL, 1.0 ml, 1.5 mL. 2.0 mL, 2.5 mL, 5.0 mL, 7.5 mL, 10 mL, 12.5 mL. 15 mL, 20 mL and 25 mL. Obviously, the prosthetic device for use in facial surgery or lip augmentation be provided in a syringe in an array of volumes, typically lower in volume than the volumes provided for by the prosthetic device for body contouring. For instance, the device for lip augmentation may be provided in volumes of 0.5 ml or 0.7 mL or 1 mL whereas the device for body contouring may be provided in volumes of 2 mL, 5 mL, or 10 mL. These are purely illustrative examples and are not intended to limit the scope of the invention in any way - the device of the invention may be provided in any volume required to perform the method.

As stated, the hydrogel is highly biocompatible. The method of the invention does not comprise of adding an antibiotic, analgesic or anti-inflammatory agent to the hydrogel.

In the preferred embodiment wherein the method comprises the injection of the endoprosthesis, the injection comprises the use of a syringe with a thin gauge needle such as a 21-29 G needle. The necessary amount of the gel is injected subcutaneously in a retrograde manner by injecting the gel while withdrawing the needle. After the injection is performed, a light manipulation may be required in order to obtain an even distribution of the gel. Post-operative oedema may be treated with local packing of ice and mild pain and redness may be experienced during the first 2-3 days after injection.

In the embodiment wherein the method comprises injection of the endoprosthesis for lip augmentation or facial correction the needle of the syringe is typically particularly thin, such as a 25-29 G needle. For body contouring, the needle of the syringe may be in the range 21-23 G.

### Polyacrylamide Hydrogel for Mammoplasty

The use of the hydrogel comprising i) less than 3.5% by weight polyacrylamide and at least 95% pyrogen-free water or saline solution for the preparation of an endoprosthesis for mammoplastic reconstruction is a preferred embodiment of this aspect of the present invention. It is most preferably implantable and further comprises a silicone-based envelope housing the hydrogel.

In the embodiment wherein the hydrogel comprises less than 3.5% by weight polyacrylamide, the hydrogel preferably comprises at least 1% by weight polyacrylamide, based on the total weight of the hydrogel, preferably at least 1.5%, such as at least 1.6% by weight polyacrylamide, based on the total weight of the hydrogel.

The hydrogels used for mammoplasty which comprise less than 3.5% by weight polyacrylamide are preferably injectable. Said injectable endoprostheses according to the present invention preferably has a viscosity complex from about 2 to 90, such as 5 to 80 Pas, preferably from about 6 to 76, such as from about 6 to 60, 6 to 40, 6 to 20, such as 6 to 15 Pas.

A further object of the invention is based on the preparation by the present investigators of a hydrogel with a higher solid-weight content. The use of a hydrogel comprising i) more than 9.5% polyacrylamide by weight, based on the total weight of the hydrogel, and ii) pyrogen-free water or saline solution for the preparation of an endoprosthesis for mammoplastic augmentation or reconstruction is a further object of the invention. Stated otherwise, a further object of the invention is a method of cosmetically altering a mammalian breast or of performing a partial or total mammoplastic reconstruction on a woman comprising implanting a polyacrylamide hydrogel endoprosthesis; wherein said hydrogel comprises more than 9.5% polyacrylamide by weight, based on the total weight of the hydrogel, and ii) at least 75% pyrogen-free water or saline solution.

In the embodiment wherein the hydrogel for mammoplasty comprises more than 9.5% polyacrylamide by weight, based on the total weight of the hydrogel, the hydrogel typically comprises less than 25% by weight polyacrylamide, based on the total weight of the hydrogel, such as less than 20%.

Endoprosthetic device for mammoplasty wherein the hydrogel comprises more than 9.5% polyacrylamide by weight are typically implantable. In said embodiments, the endoprosthesis may further comprisee a silicone-based envelope.

In the rectification of a partial or complete mastectomy, the endoprosthesis may be injectable or implantable. Wherein the endoprosthesis is implanted, the endoprosthesis may comprise a hydrogel according to the present invention in itself. The size and shape of the gel may be fixed by preparing the gel in a mould or cast or by sculpting the gel to the desired shape and size. Alternatively, prior or while implanting the gel, it may be inserted into a silicone-based shell which serves in part to contain said hydrogel.

In embodiments wherein an implantable endoprosthesis comprising a silicone-based shell is preferred, the hydrogel may be somewhat fluid or of a fixed resting shape. In embodiments wherein the endoprosthesis further comprises a silicone-based shell and the hydrogel is of a fixed resting shape, the size and shape is adapted to the requirements of the patient and may be fixed by preparing the gel in a mould or cast or by sculpting the gel to the desired shape and size. The shell may also be adapted to the size and shape of the hydrogel which it will contain. In embodiments wherein the implantable endoprosthesis does not comprise a silicone-based shell but rather that the hydrogel itself is implanted, the hydrogel is generally of a fixed resting shape. As stated, the shape and size may be adapted to the requirements of the patient and may be fixed by preparing the gel in a mould or cast or by sculpting the gel to the desired shape and size.

Wherein the endoprosthesis is injected for rectification of a partial or complete mastectomy, the endoprosthesis is preferably prepared from a hydrogel comprising 2.0 to 3.0% polyacrylamide, such as 2.2 to 3.0% polyacrylamide and having a complex viscosity of about 10 to 90 Pas, such as 20 to 90, 30 to 90, or 40 to 80 Pas and an elasticity module of about 50 to 700 Pa such as 75 to 600, such as 100 to 500, preferably about 200 to 500 Pa. Typically, the hydrogel has a complex viscosity of at least 10 Pa.s, such as at least 15 Pa s, preferably at least 20 Pa s, more preferably at least 30 Pa s, most preferably at least 40 Pa s.

### Polyacrylamide Hydrogel For The Treatment Of Incontinence And Vesicouretal Reflux

The loss of voluntary control of sphincter muscles underlies urinary and anal incontinence. Urinary incontinence, the inability to voluntarily retain one's urine in one's bladder, is common among the elderly. Contraction of the detrusor muscle of the bladder may be voluntary, brought about stress, reflex or urge. Contraction of the detrusor muscle brought about by a stress results in involuntary urination unless the bladder sphincter muscle is voluntarily contracted. In the event that one has lost control of the bladder sphincter muscle, urination may be brought about by slight stress such as slight abdominal contractions during daily activities, sneezing, coughing, laughing, gas retention, surprises and countless other stimuli which may lead to contraction of the detrusor muscle. A similar principle underlies anal incontinence wherein one has diminished control of the anal sphincter muscle. Urinary and anal incontinence may be brought upon by ageing, trauma (such as in paraplegics), or congenitally related.

Vesicouretal reflux is the result of decreased ureteral resistance wherein urine from the bladder refluxes back into the kidney. This can result in the transport of bacteria form the bladder back up through the ureter, clayceal dilation, the renal pyramids and the kidneys and may lead to infections and recurrent pyelonephritis as well as cause physiological injury to the renal parenchyma. This may lead to renal failure.

Urinary incontinence, anal incontinence and vesicouretal reflux may be treated by increasing the resistance of passage through the urethra, the colon or rectum (or *canalis analis),* and the ureter, respectively, known as bulking processes

Attempts to treat urinary incontinence have involved hydraulic apparatuses, such as in WO 01/50833 and US 4,969,474 and other controllable apparatuses such as prosthetic sphincters with inflatable cuffs as in US 4,571,749 and WO 01/47433. Weight loss, exercise, medication and surgical operation usually involving the elevation of the bladder neck or constructing a increasing resistance through the urethra using the surrounding tissue or using a prosthetic material. Materials such as collagen, PTFE, silicone, and Teflon have been used to this end (references to be added).

Anal incontinence has also been addressed via controllable and operated devices such as in WO 01/47431. Vesicouretal reflux has been dealt with surgically (to extend the ureter, usually in the infant) and with antibiotics.

A principle object of this aspect of the invention is to provide a polyacrylamide hydrogel to increase the resistance in the relevant conduits in the treatment of incontinence and vesicouretal reflux.

US 6,129,761 discloses the use of injectable hydrogel and cellular compositions towards this end. The compositions are cell suspensions comprising cells mixed with a biocompatible and biodegradable polymer. The polymer provides a medium and template for cell growth and cellular engraftment to the surrounding tissue. Cell growth coincides with polymer degradation to result in the desired tissue growth. The polymeric materials disclosed by US 6,129,761 consist of alginates such as modified alginates, bacteria! polysaccharides such as gellan gum, plant polysaccharides such as carrageenans, hyaluronic acids, polyethylene oxide-polypropylene glycol block copolymers, proteins such as fibrin, collagen, and gelatin, mixtures of polyethylene oxide and polyacrylic acid, cross-linked chitosan, photochemically cross-linked ethylenically unsaturated groups, macromers such as PEG-oligolactyl-acrylates, polyethylenimine, poly-lysine, poly(vinylamine), and poly(allylamine). US 6,129,761 does not disclose the use of stable polyacrylamide hydrogels but rather degradable poly(vinylamine). Given the requirement of the polymer of the invention of US 6,129,761 to degenerate according to the method of the invention, biostable polymers, such as the biostable polyacrylamide of the present invention is not suitable for the method of US 6,129,761.

RU2148957 relates to a method for treating cases of vesicoureteral reflux using polyacrylamide hydrogel.

The substances for injection used thus far used are synthetic substances (teflon, silicone, coal particles) and natural substances (connective tissue extract, fat). The long-term effect has been relatively poor with relapses of incontinence among approximately half the treatments after one year of observation. Thus, there is a need to find a substance which is biocompatible with tissue but at the same time is not absorbed or excreted by the body (biostable). Moreover, the substance must have adequate rheological properties to act effectively.

An object of this aspect of the invention is to provide a biocompatible and bio-stable polyacrylamide hydrogel for increasing the resistance of conduits for the treatment of incontinence and vesicouretal reflux.

The term "bio-stable" is intended to mean that the substance does not degrade to any substantial degree in interstitial fluid or body tissue.

A first aspect of this aspect of the invention relates to the bio-stable hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution, for use in the in the treatment and prevention of incontinence and vesicouretal reflux. The bio-stable hydrogel typically has a molecular weight between 0.01 x 10⁶ and 20 x 10⁶. The polymer is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble.

The present investigators have found that the bio-stable gel is effective as a bulking agent and suitable for the treatment of incontinence and vesicouretal reflux given the gel is biocompatible, non-biodegradable and does not migrate from the site of application in the conduit.

The bio-stable hydrogel of this aspect of the invention is typically obtained by combining acrylamide and methylene bis-acrylamide in a molar ratio of 150:1 to 1000:1. In a preferred embodiment, the hydrogel comprises less than 15% by weight polyacrylamide, based on the total weight of the hydrogel, preferably less 10%, more preferably less than 7.5%, even more preferably less than 5%, most preferably less than 3.5% by weight polyacrylamide, based on the total weight of the hydrogel.

Given the hydrogel of this aspect of the invention is directed for use as a permanent endoprosthesis, it must be stable. Furthermore, given the hydrogel of the invention is directed for use as an endoprosthesis for selected parts of the human anatomy, the hydrogel typically comprises at least 0.5 % by weight polyacrylamide, based on the total weight of the hydrogel, preferably at least 1.0 % by weight polyacrylamide, more preferable at least 1.5 % by weight polyacrylamide, such as at least 1.6 % by weight polyacrylamide, based on the total weight of the hydrogel. Typically, the hydrogel of the present invention may have a solid weight content of 1.5, 1.6, 1.7 1.8, 1.9, 2.0, 2.1, 2.2, 2.3, 2.4, 2.5, 2.6, 2.7, 2.8, 2.9, 3.0, 3.1, 3.2, 3.3, 3.4, or 3.5 % polyacrylamide, based on the total weight of the hydrogel.

For the treatment of vesicouretal reflux, the hydrogel preferably comprises less than 3.5% by weight polyacrylamide, based on the total weight of the hydrogel and about 96.5% pyrogen-free water of saline solution, such as 97.5% pyrogen-free water of saline solution.

The viscosity of the bio-stable hydrogel is typically such that it may be injected. In a suitable embodiment, the hydrogel has a complex viscosity of about 2 to 50 Pas, such as about 2 to 40 Pas, preferably about 2 to 30 Pas, more preferably about 2 to 20 Pas.

The device may have a viscosity such that it may be injected. In an embodiment wherein the hydrogel is injected, the hydrogel has a complex viscosity of about 2 to 30 Pa s, such as about 2 to 20 Pa s, preferably about 3 to 18 Pa s, most preferably about 3 to 15 Pa s, such as 3, 4, 5, 6, 7, 8, 9, 10, 11, 12, 13, 14, and 15 Pa s.

The device also has elastic properties due to, at lest in part of the high water binding capacity of the hydrogel of water. This is of great relevance in terms, at least, of durability and ability to provide resistance through the conduit. In a preferred embodiment, the hydrogel of the invention has an elasticity modulus of about 1 to 200 Pa, such as about 2 to 175 Pa, typically about 5 to 150 Pa, such as 10 to 100 Pa.

The elasticity modulus of the prosthetic device and the complex viscosity are typically related by a factor of 5.8 to 6.4. The present invention thus provides for a hydrogel with the advantageous combined features of a viscosity suitable for being injectable and of an elasticity to provide for increase resistance. In a combination of preferred embodiments, the hydrogel has a complex viscosity less than 25 Pa s and an elasticity modulus less than 200 Pa, preferably having a complex viscosity less than 15 Pa s and an elasticity less than 100.

Example 1, Tables 1, 2, 3 and 4 illustrate appropriate conditions for preparing the illustrative examples of hydrogels. As can be seen, within the preferred combined embodiment of a viscosity of less than 25 Pa s and an elasticity modulus of less than 200 Pa, the hydrogel may have an array of dry-weight content percentages.

Furthermore, still within the preferred combined embodiment of a viscosity of less than 25 Pa s and an elasticity less than 200 Pa, such as having a complex viscosity less than 15 Pa s and an elasticity less than 100, the hydrogel is obtainable by combining acrylamide and methylene-bis-acrylamide in a molar ratio of about 275 to 1000, typically 300 to 800, preferably in a ratio of about 300 to 500.

The hydrogel of the invention is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is an about 0.8- 1% aqueous sodium chloride solution, such as a 0.8, 0.9 or 1 % aqueous sodium chloride solution.

In a particularly suitable embodiment of this aspect of the invention, the hydrogel comprises about 2.5 % by weight polyacrylamide, based on the total weight of the hydrogel and about 97.5 % pyrogen-free water.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, typically below 5 ppm.

The washing process may suitably be performed for 15 to 250 hours, such as for 20 to 225 hours. From Table 2 and 3, it can be seen that the washing process is typically performed for 50 to 100 hours, more typically for 70 to 100 hours.

In a further embodiment of this aspect of the invention, the device prosthetic device comprises cells, such as stem cells. Polyacrylamide provides an excellent template and matrix for cell growth. The use of cells with the hydrogel of the invention for the preparation of the device would allow for cellular engraftment to the surrounding tissue in the ureter, urethra or *analis canalis.* A device comprising the hydrogel of the invention and the appropriate cells allows for greater resistance and greater efficiency in providing resistance.

In a preferred embodiment of this aspect of the invention, the hydrogel of the invention is for use in the treatment of urinary and anal incontinence, more preferably urinary incontinence.

Urinary incontinence may be stress or reflex urinary incontinence or urge urinary incontinence. Typically, the hydrogel of the invention is suitable for the treatment of stress or reflex urinary incontinence.

In a further object of this aspect of the invention, the present hydrogel is used in the preparation of an endoprosthesis. Thus, a further object of the invention is the use of a hydrogel, as described *supra,* comprising about 0.5 to 25% by weight polyacrylamide, based on the total weight of the hydrogel, for the preparation of an endoprosthesis for the treatment and prevention of incontinence and vesicouretal reflux.

The endoprosthesis is suitably formulated as an injectable suspension. The suspension comprises a homogenised formulation of the hydrogel. Typically, a syringe is filled with the suspension.

A further object of this aspect of the invention relates to a method of treating or preventing incontinence or vesicouretal reflux comprising administering a hydrogel to a mammal said hydrogel comprising 0.5 to 25% by weight polyacrylamide, based on the total weight of the hydrogel. The hydrogel, in any of the above-described embodiments, is suitable for the method of the invention.

Upon administration of the hydrogel, a thin layer of connective tissue surrounds the endoprosthesis, enabling the endoprosthesis to become a stable part of the connective tissue. Due to the stability of the hydrogel and the thin layer of connective tissue, the endoprosthesis may be removed from the patient. This advantage is at least in part due to the stability of the hydrogel which in turn is at least in part due to the washing process.

Several factors affect the rheological properties of the hydrogel, such as the relative amount of monomer used, the relative amount of initiator, the temperature and other parameters of the polymerisation process, and the washing process. Thus, the polymerisation process may provide a hydrogel with an array of viscosities. The invention is directed to an endoprosthesis typically for the urethra, the rectum or colon (or *canalis analis*), or the ureter and may thus be tailored to the requirements of the conduit.

An important object of the invention is to provide a prosthetic device for increasing the resistance of conduits selected from the group consisting of the urethra; the rectum or colon (or *canalis analis*); and the ureter for the treatment of urinary incontinence, anal incontinence, and vesicouretal reflux, respectively; wherein said device is injectable and comprising the hydrogel as described herein.

The method of this aspect of the invention preferably includes the administering of the hydrogel by means of injecting the hydrogel into the appropriate conduit. In the treatment of urinary incontinence, the hydrogel is typically injected into the urethra, specifically under the submucosal membrane of the urethra. Injection is via the external surface of the urethra and toward the submucosal membrane.

The present investigators have found that typically 2 to 5 mL of the hydrogel are suitable to provide adequate resistance in the urethra by bulking the urethra. Typically, 3 mL of hydrogel is injected and preferably the 2-5 mL are distributed by depositing the gel at more than one cross-sectional position along a single longitudinal position of the urethra. In a particularly suitable embodiment, 3 or more depots are made along a single longitudinal position of the urethra. The present investigators have found that depots 0.5 cm distally from the neck of the bladder are particularly suitable.

The present investigators have found that submucosal injections at positions 10, 2, and 6 o'clock of the cross-sectional axis of the urethra to be particularly suitable for the treatment of urinary incontinence.

The depots are typically made by means of a syringe or by use of a cytoscope or catheter. Suitably a 21 to 27G needle is employed for the injection.

For the treatment of anal incontinence, the hydrogel is typically injected into the colon or rectum (*canalis analis*) specifically under the submucosal membrane of the colon or rectum. Injections of 2 to 6 ml are suitable. The hydrogel is preferably distributed at more than one cross-sectional position along a single longitudinal position of the colon or rectum. In a particularly suitable embodiment, 3 or more depots are made along a single longitudinal position of the colon or rectum, preferably at positions 10, 2, and 6 o'clock of the cross-sectional axis of the colon or rectum.

For the treatment of vesicouretal reflux, submucosal injections into the ureter of the patient is required. Injections of 2 to 5 ml are suitable. The hydrogel is preferably distributed at more than one cross-sectional position along a single longitudinal position of the ureter In a particularly suitable embodiment, 3 or more depots are made along a single longitudinal position of the ureter, preferably at positions 10, 2, and 6 o'clock of the cross-sectional axis of the ureter.

In an alternative embodiment of this aspect of the invention, the method comprises the use of a prosthetic device comprising cells, such as stem cells. Polyacrylamide provides an excellent template and matrix for cell growth. The use of cells in combination with the hydrogel of the invention for the preparation of the device would allow for cellular engraftment to the surrounding tissue in the ureter, urethra or *analis canalis.* A method comprising the hydrogel of the invention and the appropriate cells allows for greater resistance and greater efficiency in providing resistance.

### Polyacrylamide Hydrogel For The Treatment Of Arthritis

Arthritis is a degenerative condition which, when affecting the weight bearing joints such as the hip and knee joints, results in pain and hampered mobility. Arthritis may affect all joints. Degradation of articular and meniscal cartilage may result in damage to the surfaces separated by the cartilage and correspondingly to pain. Ageing is a primary cause of the degradation of the cartilage. The degradation may also result from, for example, congenital predisposition or trauma, such as repeated articulation of the joint.

Arthritis has been treated traditionally with physiotherapy and more invasive treatments such as orthopaedic surgery and the introduction of artificial joint components. Non-steroidal anti-inflammatory agents have been used with some success but these agents may counterproductively hamper proteoglycan synthesis in collagen and cartilage as well as have undesirable side effects. Cortisone injections also weaken articular cartilage with time.

Soft compliant materials used to replace the cartilage have been developed to absorb the load on the joint and to distribute the load evenly. US 4,344,193 dislcoses silicone rubber as a prosthetic device. One difficulty with these devices is securing the devices in place and various anchoring systems have been developed (US 5,171,322; US 4,502, 161; US 4, 919, 667).

Other prosthetic devices, such as in US 5,344,459, are inflatable. The Maclntosh knee is a hard prosthetic which is painful to use.

WO 00/78356 discloses an injectable composition for promoting bone and/or cartilage growth comprising hyaluronic acid cross-linked to sulfated polysaccharides.

WO 96/24129 discloses a prosthesis for joints in hands and feet comprising a biocompatible material such as a mixture of biocompatible resin and plastics. Specific materials mentioned include polymethyl methacrylate polymer. The prosthesis is implanted into the joint. WO 00/59411 discloses a surgically implanted knee prosthesis wherein the load distribution device is constructed of material comprising a thermoset polymer or thermoplastic polymer. Hyaluronates and hyaluronic acids have been used for prosthetics and administered by injection into the intra-articular cavity of knees for the long-term relief of pain and improvement on the function of the knee joint. It has adequate viscosity and elasticity but has been prone to sheering due to mechanical stress and is biodegradable and has faced resorption problems.

There is the need in the art additional materials for use as an artificial cartilage such as in weight-bearing joints. The present invention is directed to a material and prosthetic device for use in the treatment of arthritis and to supplement or replace cartilage.

The polyacrylamide hydrogel is resistant to biological degradation and is not permeable through biological membranes. The polyacrylamide hydrogel of the invention is fully biocompatible (according to ISO standard test ISO-10993). The polyacrylamide hydrogel does not have cytotoxic effect on human fibroblasts, is non-toxic, non-carcinogenic, non-allergenic, non-mutagenic, and resistant to enzymatic and microbiological degradation. Furthermore, the polymer is not water-soluble. Notable for the invention, the polymer is resilient to mechanical stress.

The hydrogel of this aspect of the invention is for use in the in the treatment or prevention of arthritis, the hydrogel obtainable by combining acrylamide and methylene bis-acrylamide radical initiation; and washing with pyrogen-free water or saline solution, the combining being in amounts and the washing being such that to give about 0.5 to 25% by weight polyacrylamide, based on the total weight of the hydrogel. The hydrogel obtained has been shown by the present investigators to be both biostable and biocompatible, and is not resorbed by the body. Moreover, the present investigators have demonstrated the hydrogel to be resilient to mechanical stress.

Typically, the hydrogel of this aspect of the invention is obtained by combining acrylamide and methylene bis-acrylamide is in a molar ratio of 150:1 to 1000:1. The conditions for obtaining the hydrogel may be modified according to, for instance, the nature of the joint into which the hydrogel is intended to be injected. The desired rheologically properties, such as elasticity and viscosity may be controlled at least in part by the solid weight content of the hydrogel. The hydrogel of the invention comprises about 0.5 to 25% by weight polyacrylamide, based on the total weight of the hydrogel. In suitable embodiments of the invention, the hydrogel comprises less than 15% by weight polyacrylamide, based on the total weight of the hydrogel, preferably less 10%, more preferably less than 7.5%, even more preferably less than 5%, most preferably less than 3.5% by weight polyacrylamide, based on the total weight of the hydrogel.

Given the hydrogel of this aspect of the invention is directed for use as an endoprosthesis, it must be stable. Typically, such as for reasons of increased stability, the hydrogel comprises at least 1% by weight polyacrylamide, based on the total weight of the hydrogel, preferably at least 1.5%, such as 1.6% by weight polyacrylamide, based on the total weight of the hydrogel. In suitable embodiments, the hydrogel of the present invention has a solid weight content of at least 1.5 and less than 3.5% by weight polyacrylamide, based on the total weight of the hydrogel such as 1.5, 1.6, 1.7 1.8, 1.9, 2.0, 2.1, 2.2, 2.3, 2.4, 2.5, 2.6, 2.7, 2.8, 2.9, 3.0, 3.1, 3.2, 3.3, and 3.4% polyacrylamide, based on the total weight of the hydrogel.

The combining involves the combining of the component reagents acrylamide and methylene bis-acrylamide, typically degassed and typically in a manner to minimise operator contact. The reagent components may be optionally previously combined to form an inert mixture. An inert mixture is one wherein no chemical reaction proceeds among the component reagents. The combining involves combining acrylamide, methylene-bis-acrylamide, and a radical initiator component. In a suitable embodiment, an inert premixture of acrylamide, methylene-bis-acrylamide (the cross-linker) and TEMED is combined with an AMPS initiator solution. However, the components may be combined as singularities or as alternative plural premixtures.

Acrylamide and methylene-bis-acrylamide are suitably combined in a molar ratio of about 100:1 1 to 1000:1, typically about 150:1 to 900:1, preferably about 175:1 to 800:1, more preferably about 200:1 to 600:1, most preferably from 250:1 to 500:1. As shown in Tables 2 and 3, hydrogels of differing solid-weight content and rheological properties may be controllably prepared. The hydrogel having the desired rheological characteristics has been obtained by combining acrylamide and methylene-bis-acrylamide in a ratio of about 250:1, about 260:1, about 270:1, about 280:1, about 290:1, about about 300:1, about 310:1, about 320:1, about 330:1, about 340:1, about 350:1, about 360:1, about 370:1, about 380:1, about 390:1, about 400:1, about 410:1, about 420:1, about 430:1, about 440:1, about 450:1, about 460:1, about 470:1, about 480:1, about 490:1 and about 500:1.

As can also be seen from Tables 2 and 3, the relative amount of monomer (acrylamide and methylene-bis-acrylamide) is fairly constant from formulation to formulation in relation to TEMED. Thus, in a preferred embodiment of the method of the invention, the ratio of monomers to TEMED is relatively constant from batch to batch and not used to regulate the rheological properties of the polymer. In the embodiment wherein the polymer is polyacrylamide, the ratio of the monomers acrylamide and methylene-bis-acrylamide to TEMED is about 100:1 to 700:1, such as 200:1 to 600:1, typically 200:1 to 500:1, preferably 200:1 to 400:1, most preferably 200:1 to 350:1.

Similarly, the relative amount of monomer (acrylamide and methylene-bis-acrylamide) is fairly constant from formulation to formulation in relation to the amount of initiator. Thus, in a preferred embodiment of the method of the invention, the ratio of monomers to initiator is relatively constant from batch to batch and not used to regulate the rheological properties of the polymer. In the embodiment wherein the polymer is polyacrylamide, the ratio of the monomers acrylamide and methylene-bis-acrylamide to initiator is about 100:1 to 700:1, such as 200:1 to 600:1, typically 200:1 to 500:1, preferably 200:1 to 400:1, most preferably 200:1 to 350:1.

The viscosity of the hydrogel is suitably such that it may be injected. In a typical embodiment, the hydrogel has a complex viscosity of about 2 to 20 Pa s, such as about 3 to 18 Pa s, preferably about 3 to 15 Pa s, most preferably about 2 to 13 Pa s.

The hydrogel of the invention is substantially free of materials which contribute to the solid weight content other than the acrylamide, methylene-bis-acrylamide and residual amounts (if any) of the initiators. The hydrogel is substantially free of any other polymeric content. The hydrogel further comprises at least 75% by weight pyrogen-free water or saline solution, preferably pyrogen-free water. In a suitable embodiment of the invention, the hydrogel comprises at least 80% by weight pyrogen-free water or saline solution, preferably at least 85 %, more preferably at least 90%, even more preferably at least 95% by weight pyrogen-free water or saline solution.

A suitable saline solution has an osmolarity similar to that of interstitial fluid. Suitable saline solutions include but are not limited to the group selected from 0.25- 1 % aqueous sodium chloride, a Ringer-Lockart solution, an Earle solution, a Hanks solution, an Eagle medium, a 0.25 - 1 % glucose solution, a potassium chloride solution, and a calcium chloride solution. In a preferred embodiment, the saline solution is a 0.8-1 % aqueous sodium chloride solution, such as a 0.8, 0.9 or 1 % aqueous sodium chloride solution, most preferably about 0.9 % aqueous sodium chloride.

As will be obvious to the person skilled in the art, in the embodiment wherein saline solution is used either for the preparation of the gel and/or for the washing of the gel, the solid-weight content of the gel will be higher than the contribution made by the polyacrylamide, but typically not more than an additional 1 %.

In a particularly suitable embodiment of the invention, the hydrogel comprises about 2.5 % by weight polyacrylamide, based on the total weight of the hydrogel and about 97.5 % pyrogen-free water.

Pyrogen-free water or saline solution is used for the washing process. The washing process serves, in part, to remove all but trace amounts of the monomers acrylamide and N,N'-methylene-bis-acrylamide. These monomers are toxic to the patient as well as detrimental to the stability of the hydrogel. The washing process is preferably such that the concentrations of the monomers acrylamide and N,N'-methylene-bis-acrylamide are below 50 ppm, more preferably below 40 ppm, such as below 30 ppm, most preferably below 20 ppm, typically below 10 ppm, particularly preferably below 5 ppm.

In an alternative embodiment of the invention, the hydrogel has a more solid consistency such that it is implantable into a joint cavity. In such embodiments wherein the hydrogel has a solid consistency, the hydrogel may be surface-modified so as to decrease slippage from the area it was implanted. Surface modification may be chemical or physical in nature. Hydrogels of with a solid weight content have been prepared which are solid like and suitable for implantation rather than injection and very amenable to surface modification.

In the embodiment wherein the hydrogel is implantable, the viscosity of the solid sample is obviously very high. In the embodiment wherein the hydrogel is implantable, the hydrogel has a complex viscosity of about 20 to 1500 Pa s, typically 20 to 1000 Pa s.

In the embodiment of this aspect of the invention wherein the hydrogel is implantable and furthermore surface-modified, in the event that the hydrogel is surface modified by chemical treatment, it is anticipated that the chemical treatment will account for less than 1 % of the weight of the hydrogel based on the total weight of the hydrogel. Chemical treatment may comprise of a surface coating agent or an agent which acts topically to chemically modify the polyacrylamide at the surface of the hydrogel.

The hydrogel of this aspect of the invention is used for the preparation of an endoprosthesis to be inserted into the intra-articular cavity of joint. The gel is intended for use as a prosthetic device in the treatment of arthritis. The prosthetic device of the invention comprises a polyacrylamide hydrogel comprising 0.5 to 25% by weight polyacrylamide, based on the total weight of the hydrogel and is administered to the intra-articular cavity of joint.

The prosthetic device may comprise any embodiment of the hydrogel as described supra. According, the device is for augmenting or replacing cartilage in the intra-articular cavity of a joint, said device comprises a polyacrylamide hydrogel comprising 0.5 to 25% by weight polyacrylamide, based on the total weight of the hydrogel. The prosthetic device of the invention the hydrogel typically further comprises at least 75% by weight pyrogen-free water or saline solution, preferably pyrogen-free water. It may be administered by implantation or injection into the intra-articular cavity of a joint. Preferably, the device is injected.

The device may have a viscosity such that it may be injected. In an embodiment wherein the hydrogel is injected, the hydrogel has a complex viscosity of about 2 to 25 Pa s, such as about 3 to 20 Pa s, preferably about 3 to 18 Pa s, most preferably about 3 to 15 Pa s, such as 3, 4, 5, 6, 7, 8, 9, 10, 11, 12, 13,14, and 15 Pa s.

Particularly in the embodiment wherein the hydrogel is injected into a weight-bearing joint, the elasticity of the hydrogel and device is of great relevance. In a preferred embodiment, the hydrogel of the invention has an elasticity modulus of about 1 to 200 Pa, such as about 2 to 175 Pa, typically about 5 to 150 Pa, such as 10 to 100 Pa.

The elasticity modulus of the prosthetic device and the complex viscosity are typically related by a factor of 5.8 to 6.4. The present invention thus provides for a hydrogel with the advantageous combined features of a viscosity suitable for being injectable and of an elasticity to supplement weight-bearing capacity. In a combination of preferred embodiments, the hydrogel has a complex viscosity less than 25 Pa s and an elasticity modulus less than 200 Pa, preferably having a complex viscosity less than 15 Pa s and an elasticity modulus less than 100 Pa.

Example 1, Tables 1, 2 and 3, illustrate appropriate conditions for preparing the illustrative examples of hydrogels. As can be seen, within the preferred combined embodiment of a viscosity of less than 25 Pa s and an elasticity modulus of less than 200 Pa, such as a complex viscosity of less than 15 Pa s and an elasticity of less than 100, the hydrogel may have an array of dry-weight content percentages.

Furthermore, still within the preferred combined embodiment of a viscosity of less than 25 Pa s and an elasticity less than 200 Pa, such as having a complex viscosity less than 15 Pa s and an elasticity less than 100, the hydrogel is obtainable by combining acrylamide and methylene-bis-acrylamide in a molar ratio of about 275 to 1000, typically 300 to 800, preferably in a ratio of about 300 to 500.

The hydrogel of the invention is resilient to mechanical stress as well as prividing lubrication within the joint. This mimics the combination of naturally occurring cartilage and synovial fluid in the joint. Synovial fluid is produced by the synovial membrane and forms in the interface with both the synovium and the articular cartilage. Its function is nutrition of the cartilage, lubrication, load bearing and shock absorption (see Gomez and Thurston, Biorheology 30, 409-427 (1993)). Synovial liquid is a solution of a very sophisticated polymer complex made of the linear hyaluronic acid backbone having protein branches and has an elasticity modulus G' of 60 Pa and a complex viscosity of about 1-10 Pa s. Certain embodiments of the hydrogel of the invention typically has very similar elasticity modulus and viscosity.

Synovial liquid, being a fluid, has a very long relaxation time (about 100 sec). The relaxation time is defined as the time required for the stress to decay to 37% of its initial value in a stress relaxation experiment. This long relaxation time implies that for fast movements (when the stress is applied rapidly), it responds as a very elastic material, whereas at low speed stresses it behaves like a lubricating oil. Low viscosity formulations of the hydrogel of the invention are fluid-like, such as when homogenised, and as fluids, have the very long relaxation time and the fluid like properties of synovial liquid. The present invention thus provides for an excellent alternative to known technologies for replacing synovial liquid, such as injections with isotonic (NaCl) water which lasts for only 1-3 days, or injection with solutions of hyaluronic acid, which faces resorption problems.

As stated, in embodiments wherein the hydrogel is as a low viscosity formulation, the hydrogel attempts to mimic, at least in part, the features of synovial liquid. In embodiments where the hydrogel is of higher viscosity formulation, such as a viscosity above 10 Pa s, such as above 15 Pa s, the hydrogel mimics more the combined features of synovial liquid and cartilage in that the hydrogels are more elastic materials with infinite relaxation times.

In terms of resilience to mechanical stress, the hydrogel of the invention has demonstrated complete full resilient behaviour. Moreover, the elastic feature of the hydrogel allows it to return to its start position when the stress is released in creep experiments.

The device may be administered into an array of intra-articular cavities where said joint or cartilage present in said joint may need increased lubrication, increased weight bearing capacity, or increased protection of the opposing bones of the joint, such as but not limited to the group comprising of the knee joint; hip joint; the elbow, the metacarpal-phalangeal and interphalangeal joints in hands and feet (please comment as to other relevant joints).

In the administration of the device to the patient, the adequate placing and position of the hydrogel is of great relevance. To assist in the positioning of the gel, visualisation of the location of the device is useful to the individual performing the procedure. It may be advantageous, such as in the embodiment wherein the device is administered by injection to visualise the device in order to establish its position and the amount required. Visualisation of the hydrogel during administration may be facilitated by radio-labelling of the hydrogel. Thus, in one interesting embodiment, the hydrogel is radio-labelled.

As stated, surface treatment of the hydrogel may assist device to be held in place within the joint cavity. However, in the embodiment of an injectable hydrogel, the device may be held by the bone tissue which defines the boundaries of the cavity into which the gel was injected.

As also stated, one aspect of the invention relates to the use of a hydrogel comprising about 0.5 to 25% by weight polyacrylamide, based on the total weight of the hydrogel, for the preparation of an endoprosthesis for alleviation or prevention of symptoms associated with arthritis. Correspondingly, the method of the invention may be defined as a method of treating or preventing arthritis comprising administering a hydrogel to a mammal said hydrogel comprising 0.5 to 25% by weight polyacrylamide, based on the total weight of the hydrogel. The method comprises the use of the hydrogel as defined supra and to the use of the prosthetic device as defined supra.

The method may comprise a first series or treatment session of injections or implantations followed by an analysis, evaluation or trial of the degree of assistance provided by the device and followed by further series or treatment sessions if required. In the embodiment wherein the joint required primarily increased weight bearing capacity, the elasticity or support provided by the device may diminish with time. However, one advantage of the method of the invention is that an injectable formulation of the prosthetic device may be administered to supplement the existing device. This procedure may be repeated as often as required by the patient to alleviate the pain associated with the condition. Similarly, in the event that the device was administered primarily to increase lubrication in the joint, and that the lubrication capacity of the device has diminished with time, the method of the invention, such as by simple injection may be repeated as required.

### Polyacrylamide Hydrogel for Phalloplasty

A further object of the invention is the use of a hydrogel comprising i) less than 1.6% polyacrylamide by weight, based on the total weight of the hydrogel for the preparation of an endoprosthesis for penis enlargement. This method of augmenting the size of a penis comprising the administration of a polyacrylamide hydrogel, wherein the hydrogel comprises less than 3.5% polyacrylamide by weight, based on the total weight of the hydrogel, is typically performed by means of an injection. The administration by means of injection is made into cavernous tissue.

The hydrogel typically further comprises at least 95% pyrogen-free water or saline solution. Thus, a particularly interesting embodiment comprises the use of a hydrogel comprising i) less than 1.6% polyacrylamide by weight, based on the total weight of the hydrogel and ii) at least 95% pyrogen-free water or saline solution for the preparation of an endoprosthesis for penis enlargement, preferably an injectable endoprosthesis. Injectable endoprostheses for penis enlargement typically have a viscosity complex of less than 20 Pa s, such as less than 18 Pa s, preferably less than 15 Pa s. Moreover said endprostheses typically have an elasticity module of less than 150 Pa, typically less than 100 Pa.

A still further object of this aspect of the invention is the use of a hydrogel comprising i) more than 9.5% polyacrylamide by weight, based on the total weight of the hydrogel, and ii) pyrogen-free water or saline solution for the preparation of an implantable endoprosthesis for penis enlargement. This method of augmenting the size of a penis comprising the implantation of a polyacrylamide hydrogel endoprosthesis wherein the hydrogel comprises i) more than 9.5% polyacrylamide by weight, and ii) pyrogen-free water or saline solution may be performed by implantation or injection of the endoprosthetic device.

The hydrogel of the prosthetic device for penile augmentation comprising at least 9.5% polyacrylamide has a complex viscosity of at least 10 Pa s, such as at least 15 Pa s, preferably at least 20 Pa s, more preferably at least 30 Pa s, most preferably at least 40 Pa s. The high viscosity gels are suitable for implantation and have a semi-flacid to semirigid solid consistency, appropriate for this purpose.

### Polyacrylamide Hydrogel for Treating Oesophagitis

A further object of the invention is the use of a hydrogel comprising i) more than 6% polyacrylamide by weight, based on the total weight of the hydrogel, and ii) pyrogen-free water or saline solution for the preparation of an endoprosthesis for treating (reflux) oesophagitis. In a suitable embodiment, the hydrogel comprises more than 7, 8, or 9% polyacrylamide. This method for treating (reflux) oesophagitis comprising implanting or injecting a polyacrylamide hydrogel endoprosthesis wherein the hydrogel comprises more than 6% polyacrylamide by weight, based on the total weight of the hydrogel may be performed by implantation or injection of the endoprosthetic device, typically be injection or implantation of into the submucosal layer of the tissue.

A still further object of the invention is the use of a hydrogel comprising i) less than 3.5% polyacrylamide by weight, based on the total weight of the hydrogel, and ii) pyrogen-free water or saline solution for the preparation of an endoprosthesis for treating (reflux) oesophagitis. This method for treating (reflux) oesophagitis may be by implanting or injecting an hydrogel wherein the hydrogel comprises less than 3.5% polyacrylamide by weight, based on the total weight of the hydrogel. In the treatment of (reflux) oesophagitis using a hydrogel comprising i) less than 3.5%, the hydrogel typically further comprises at least 95% water or saline solution and is typically performed by injection. In these embodiments, the degree of cross-linking may be such that the viscosity is at least 6.0 Pas, such as from 6.0 to 90 Pas, such as from 10 to 80 Pas, preferably from 20 to 80 Pas. The degree of cross-linking of the hydrogel used for the preparation of an endoprosthesis for treating reflux oesophagitis may be such that the elasticity module is from about 50 to 700 Pa such as 75 to 600, such as 100 to 500, preferably about 200 to 500 Pa. Typically, the hydrogel has a complex viscosity of at least 20 Pa s, more preferably at least 30 Pa s, most preferably at least 40 Pa s.

The invention is further disclosed by the Examples which are not intended to be limiting in any way.

### EXAMPLES

### Example 1

### Preparation of Hydrogel

The gel is a polyacrylamide gel manufactured by a polymerisation of the monomers of acrylamide and N,N'-methylene-bis-acrylamide. The finished product may have different viscosities.

The hydrogel has the empirical formula [C₃H₅NO]ₓ[C₇H₁₀N₂O₂]_{y} and the structural formula as shown in Figure 1

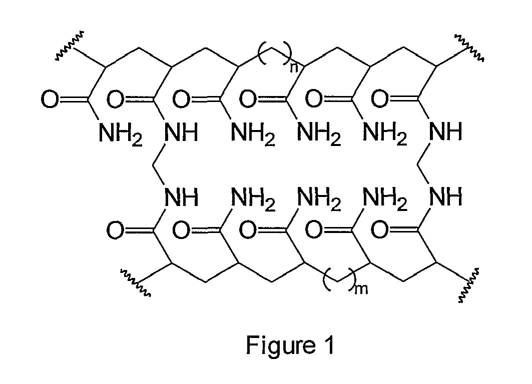

The hydrogel typically contains approximately 95% water. The concentration of the monomers acrylamide and N,N'-methylene-bis-acrylamide has been shown to be less than 10 ppm and is adequate for the desired stability of the final product, often less than 5 ppm.

The finished product must conform with respect to pH, absence of heavy metals, refractive index, stability, absence of pyrogens, and must be sterile, practically inert, and be substantially free of monomers.

### Preparation 1.1

The synthetic preparation suitably involves the following operations:
1. Two mixtures, A1 and A2, are prepared. A1 comprises water, acrylamide, N,N'-methylene-bis-acrylamide, N,N,N',N'-tetramethylene-ethylene-diamine (TEMED). A2 comprises water and ammonium persulphate;
2. The two mixtures are combined in the following ratio: 1990 mL of A1 and 10 mL of A2 and kept at 45 °C and degassed with nitrogen for 20 seconds;
3. The reaction mixture is cast into several 100 mL beakers;
4. Polymerisation is allowed to occur for 0.5 to 1.5 hours;
5. The gel is demolded;
6. Residual monomers are extracted and with equilibration in WFI water for 92 hours, changing the water several times, typically 8 times during the 92 hours;
7. The purified gels are homogenised by grinding with an vertically oscillating grid;
8.The syringe is filled with the homogenised gel material;
9. Autoclavation of the syringe

A typical method for preparing the hydrogel may be summarised as:

### Preparation 1.2

*Process summary*: The gel is prepared by mixing an aqueous monomer solution of acrylamide (AM) and N,N'-methylene-bis-acrylamide (BISAM) as cross-linker with N,N,N',N'-tetramethylene ethylene diamine (TMED) as co-initiator and ammoniumpersulfate (APS) as free-radical initiator (redox-system). By degassing a bulk solution with nitrogen polymerisation starts. After final polymerisation the gel transferred into a washing tank with net trays onto which the gel is placed. During water washing the gel swells and monomer residues are extracted. The swollen gel is fed and evacuated in a filling unit having the gel delivered in a syringe, which is autoclaved.

Two alternate formulations have been prepared, a lower- and a higher- end viscosity formulation. Both formulations have a solid weight content of less than 3.5% and a complex viscosity in the range of 2 to 50 Pa s, typically between 3 and 20 Pa s.

**Table 1**

| Chemical constituent | lower end viscosity | higher end viscosity |
|---|---|---|
| acrylamide | 502 g | 547 g |
| N,N'-methylene-bis-acrylamide | 2,2 g | 4,6 g |
| TMED | 3,0 g | 2,6 g |
| APS | 5,4 g | 5,0 g |
| Non-pyrogenic water | Add 10 litre | Add 10 litre |

The above are typical preparations of the hydrogel and may be adjusted within certain ranges.

### Preparation 1.3

### Polyacrylamide formulations from inline cross-linking process

A particularly interesting method of preparing the hydrogels of the invention involves an inline cross-linking process. Two individual and eventually degassed flows, one being a pre-mix of acrylic amide, bis-methylene acryl amide (the cross-linker) and TEMED, the other being the AMPS initiator solution, are pumped into a static mixer for mixing, chemical initiation and subsequent extrusion downstream into a pipe reactor made of Teflon or steel in which the polymerisation occurs. Washing of the gel is simplified due to high surface area of gel from reactor.

By selecting monomer, cross-linker and initiator concentrations and their relative molar ratios, and by regulating the two flow rates and the polymerisation temperatures, it is possible to produce gels that are varying in degree of crosslinking and in solids content.

### Preparation 1.4

The reagents were combined in ratios described in Tables 2, 3 and 4, and washed as described in the Tables (with pyrogen-free water unless indicated otherwise) to give low, medium, and high viscosity formulations. Hydrogels with solid weight contents between 0.5 and 25% polyacrylamide were prepared.

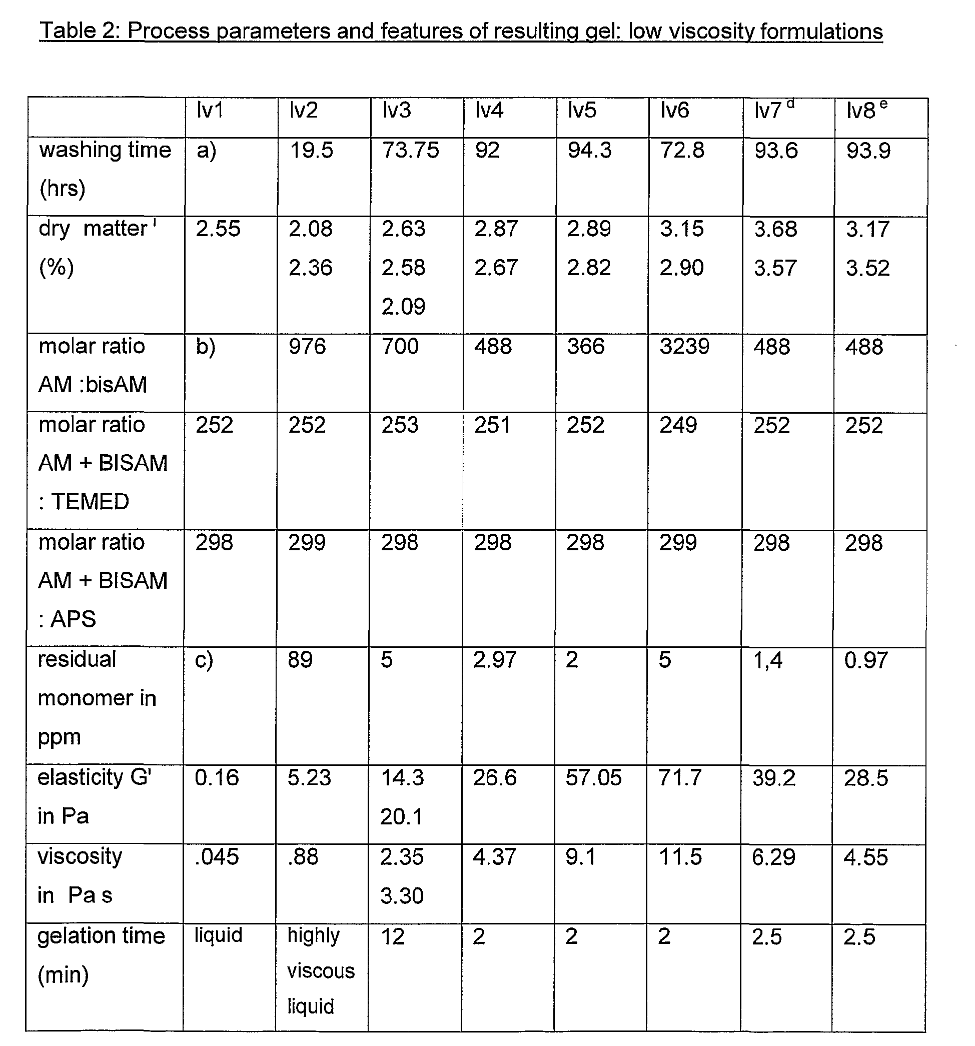

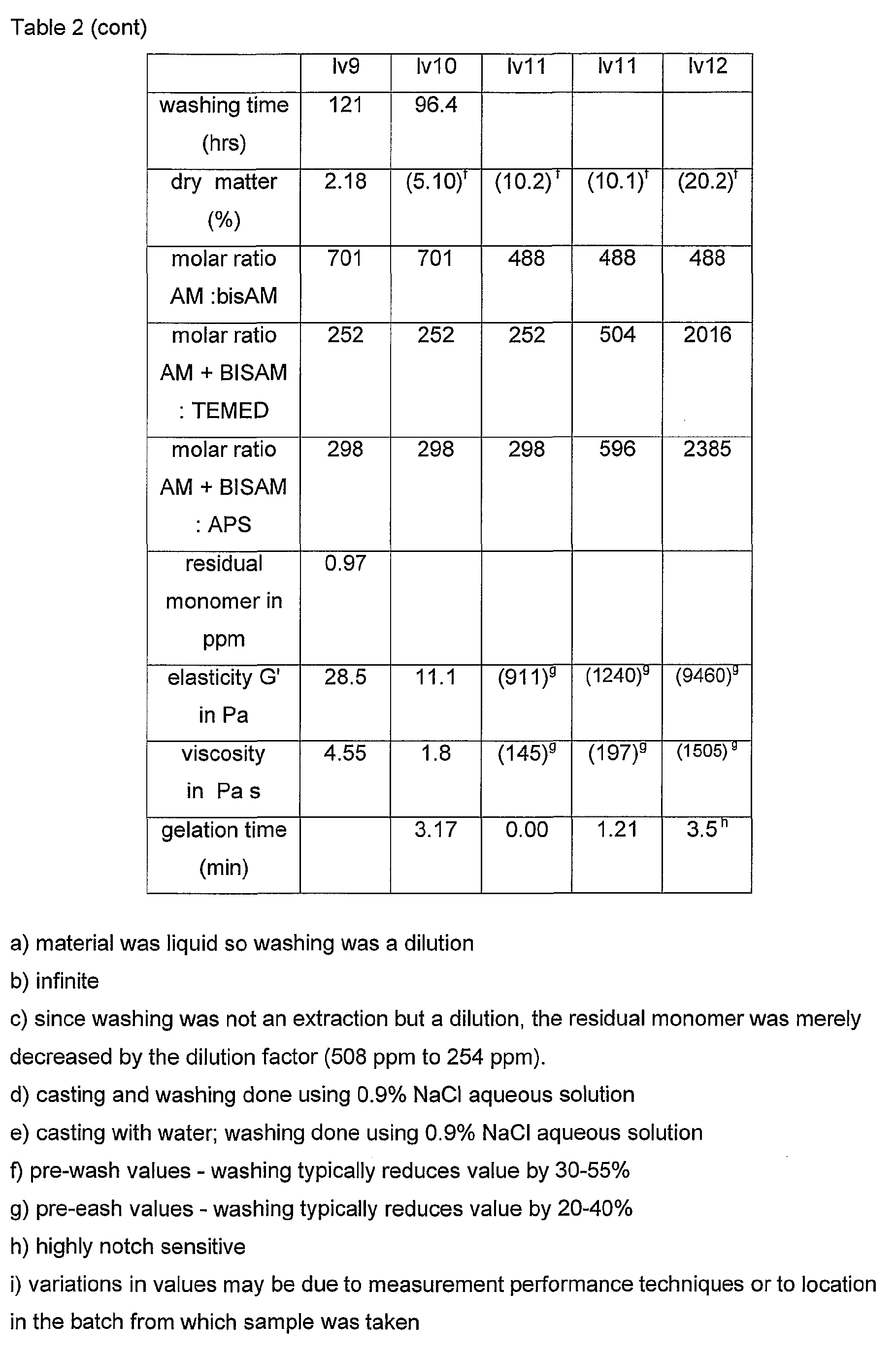

**Table 3: Process parameters and features of resulting gel: medium viscosity formulations**

| | mv1 | mv2 | mv3 | mv4 | mv5 |
|---|---|---|---|---|---|
| washing time (hrs) | 97 | 211.5 | 96 | 94.8 | 90.3 |
| dry matter (%) | 3.14 | 2.49 | 3.25 | 3.29 | 3.22 |
| molar ratio AM :bisAM | 310 | 310 | 290 | 289 | 289 |
| molar ratio AM + BISAM :TEMED | 252 | 252 | 252 | 251 | 252 |
| molar ratio AM + BISAM : APS | 299 | 299 | 299 | 299 | 299 |
| residual monomer in ppm | 1.6 | | 1.5 | | |
| elasticity G' in Pa | 108.5 | | 129 | 133.5 | |
| viscosity in Pa s | 17.4 | | 20.6 | 21.30 | |
| gelation time (min) | 2.5 | 2.5 | 2.18 | | |

**Table 4: Process parameters and features of resulting gel: high viscosity formulations**

| | hv1 | hv2 | hv3 | hv4 | hv5 |
|---|---|---|---|---|---|
| washing time (hrs) | 119.5 | 516 | 122 | 95.5 | 116.7 |
| dry matter (%) | 3.47 | 2.5 | 3.56 | 3.83 | 3.42 |
| molar ratio | 260 | 260 | 260 | 260 | 260 |
| AM :bisAM | | | | | |
| molar ratio AM + BISAM : TEMED | 315 | 315 | 604 | 313 | 314 |
| molar ratio AM + BISAM : APS | 376 | 376 | 755 | 375 | 376 |
| residual monomer in ppm | 0.2 | | | | |
| elasticity G' in Pa | 343 | 274 | | 314.5 | |
| viscosity in Pa s | 54.7 | 43.65 | | 50.1 | |
| gelation time (min) | 2.18 | 2.18 | 7.5 | | |

### Example 2

### Analysis of Hydrogel

### Characteristics of the gel

The washed hydrogels will have typical properties outlined below.

**Table 5**

| Property | Low viscosity version | High viscosity version |
|---|---|---|
| Cross-linking density | 0,25% | 0,40% |
| Swelling rate | 80-120% | 50-70% |
| Rheol. elasticity modulus, G' | 10-80 Pa | 250-700 Pa |
| Rheol. Complex viscosity, η* | 2-10 Pas | 50-90 Pas |
| Dry matter | 1,6-3% | 3-5% |
| Refractive index | 1,335-1,338 | 1,338-1,340 |

### Example 3

Swelling occurs during wash procedure and will typically show a swelling profile as shown in below Figures 2 and 3.

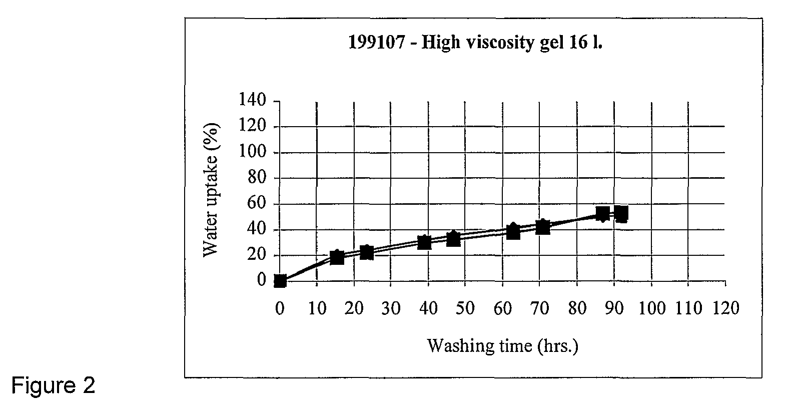

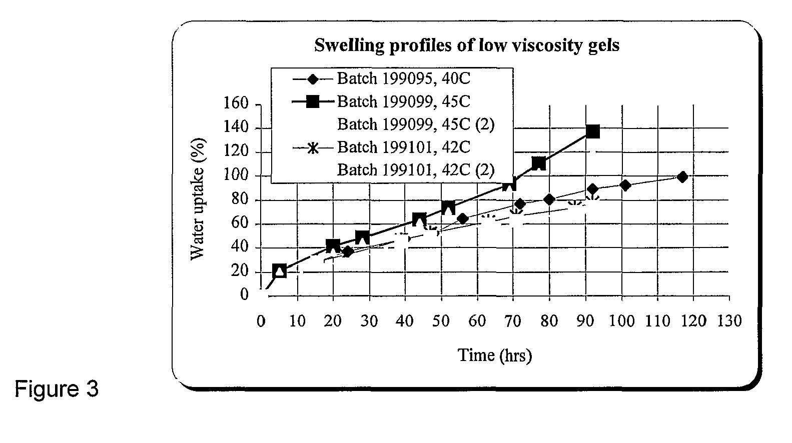

### Example 4

### Method of Administration for Reflux Oesophagitis

### Treatment by injection

The polyacrylamide hydrogel (solid weight content 2.5% and ca. 97.5% pyrogen-free water) is injected under the mucous membrane of the trachea or connecting conduits from the stomach so as to provide increased density of the conduit. It is carried out by during short procedure involving few complications.

### Example 5

### Method of Administration for Body Contouring

### Treatment by injection

a) The injection of the gel may be performed under local anaesthesia.
b) The procedure must be performed under sterile conditions. Pharmaceuticals are not to be injected into the gel
c) The gel is pre-filled in sterile syringes of 1 mL with luer-lock and should be injected subcutaneously with a thin gauge needle, e.g 27 G. Needles should be CE-marked.
d) Inject the necessary amount of the gel subcutaneously in a retrograde manner by injecting the gel while withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Treatment by implantation

The gel, optionally in sealed in a silicone-based envelope is placed subcutaneously into the soft tissue of the patient.

### Example 6

### Method of Administration for Reflux Oesophagitis

### Treatment by injection

The polyacrylamide hydrogel (solid weight content 2.5% and ca. 97.5% pyrogen-free water) is injected under the mucous membrane of the conduit between the oesophagus and stomach, such as to re-inforce the sphincter so as to provide increased density of the conduit. It is carried out by during short procedure involving few complications.

### Example 7

### Method of Administration for Body Contouring

### Treatment by injection

a) The injection of the gel may be performed under local anaesthesia.
b) The procedure must be performed under sterile conditions. Pharmaceuticals are not to be injected into the gel
c) The gel is pre-filled in sterile syringes of 1 mL with luer-lock and should be injected subcutaneously with a thin gauge needle, e.g 27 G. Needles should be CE-marked.
d) Inject the necessary amount of the gel subcutaneously in a retrograde manner by injecting the gel while withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Treatment by implantation

The gel, optionally in sealed in a silicone-based envelope is placed subcutaneously into the soft tissue of the patient.

### Example 8

### Method of Administration for Soft Tissue Filling

a) The injection of the gel may be performed under local anaesthesia, but for correction of wrinkles and folds, local anaesthesia is not necessarily required. For lip augmentation, anaesthesia through the nerve block is recommended.
b) The procedure must be performed under sterile conditions. Pharmaceuticals are not to be injected into the gel
c) The gel is pre-filled in sterile syringes of 1 mL with luer-lock and should be injected subcutaneously with a thin gauge needle, e.g 27 G. Needles should be CE-marked.
d) Inject the necessary amount of the gel subcutaneously in a retrograde manner by injecting the gel while withdrawing the needle. A patient record label is part of the packaging and is removable and to be attached to the patient record to ensure that the product is traceable.
e) After the injection is performed, a light manipulation may be performed in order to obtain an even or desired distribution of the gel. The injected gel will form a stable, soft part in the connective tissue and will give long lasting cosmetically satisfactory appearance.
f) Further injection sessions may be performed to achieve the desired affect.

### Post-operative procedures

If oedema occurs, ice packs may be applied locally. Exposure to direct sunlight or extreme cold or heat is advised until the initial swelling and redness has been resolved.

### Adverse Events/Side Effects

It is not uncommon for patients to develop some pain within the first 2-3 days postoperatively. A mild degree of oedema will occur in some patients during the first 2-3 days after injection.

The correct injection technique is crucial for the final result of the treatment and to be performed by authorised personnel.

The gel is sterilised (such as by moist heat or autoclavation). In the even that the package is damaged or opened but unused, sterility may be compromised and the contents should be discarded. Re-sterilisation is not advisable.

### Example 9

### Clinical Experience for Soft Tissue Filling

1) Approximately 900 patients underwent facial corrections with the gel. The overall cosmetic results were excellent and the frequency or adverse events was 0.02% (Kovanskaya V.A.; Scienctific conference, 13-16 October 2000).
2) A total of 150 adults undergoing correction of the contour deformities of the face were treated with the injectable gel. The amount of gel injected was from 0.2 to 11 mL.

Scheduled visits took place at a screening day (3 days prior to day 0), day 0 (first injection), day 7, day 28, month 3, month 6 and at end of study visit month 12 and underwent a physical examination and vital signs test, pregnancy test, blood and serum analysis, hematology test, immunology test, urine analysis, concomitant treatment, side effect and events analysis, cosmetic outcome analysis by patient and surgeon as well as completing a questionnaire according to the schedule of Table 6.

### Results

The overall rating of the outcome of the surgery was from very good to good from both the patients and the surgeon. In some instances, the patients wanted to continue the treatment and receive further injections. Several surgeons remarked spontaneously on the questionnaire that the patients were happy with the result and that the gel was easy to handle and administer.

The gel was very well tolerated. Only a few side-effects were recorded and the Adverse Events oedema and inflammation were reported by the patient. The Adverse Events resolved spontaneously after a few days.

**Table 6**

| | Screening (at least -3 days) | Day 0 (Facial correction) | | Day 7 ±1 day | Day 28 ±2 days | month 3 ±7 days | month 6 ±7 days | month 12 ±7 days |
|---|---|---|---|---|---|---|---|---|
| | | pre-op | post-op | | | | | |
| information / informed consent | X | | | | | | | |
| physical examination | | X | | X | X | X | X | X |
| vital signs | | X | | | | | X | X |
| pregnancy test | | X | | | | | | |
| blood and serum analysis | **sampling** | X | | | | | X | X |
| Haematology | **sampling** | X | | | | | X | X |
| Immunology | **sampling** | X | | | | | X | X |
| Urine analysis | | X | | | | | X | X |
| Concomitant treatment | | X | X | X | X | X | X | X |
| side effects and events | | | X | X | X | X | X | X |
| questionnaire (complaints) | | | X | X | X | X | X | X |
| Cosmetic outcome Patient Surgeon | | | | | | | | |
| | | | | | X | X | X | X |
| | | | | | X | X | X | X |

### Example 10

### Method of Administration and Clinical Results for Treating Incontinence

### Treatment by injection

The polyacrylamide hydrogel (solid weight content 2.5% and ca. 97.5% pyrogen-free water) is injected under the mucous membrane of the urethra so as to provide increased density of the urethra is carried out by during short procedure involving few complications.

The medical procedure involves injection of polyacrylamide gel under the mucous membrane of the urethra of women suffering from incontinence. Injection is via the external surface of the urethra and toward the submucosal membrane. 3 mL are injected at three depots sites are made along a single longitudinal position of the urethra. Depots 0.5 cm distally from the neck of the bladder were made.

The injections are carried out under a local anaesthetic, enabling the bladder to be filled subsequently and the patients to cough in order to establish immediately whether sufficient density for urinary incontinence has been achieved. On lack of effect, the injection may be repeated which is common in connection with treatment by injection.

Examination of the woman found the treatment by injection suitable according to the standards and regimes of the Gynaecology- Obstetrics department of Copenhagen's Amstsygehus (County Hospital) in Glostrup. Results and any complications are monitored at check-up examinations every three months for one year after the treatment.

## Claims

1. A hydrogel for use in the treatment of arthritis,
said hydrogel obtainable by combining acrylamide and methylene bis-acrylamide in amounts so as to give about 0.5 to 25% by weight polyacrylamide, based on the total weight of the hydrogel; radical initiation; and washing with pyrogen-free water or saline solution.

2. The hydrogel according to claim 1, wherein the hydrogel is administered by injection into the intra-articular cavity of a joint.

3. The hydrogel according to claim 1, wherein the arthritis is in the knee joint, hip joint, the elbow, or metacarpal-phalangeal and interphalangeal joints in hands and feet.

4. The hydrogel according to any one of the preceding claims, wherein the hydrogel has an elasticity module of from 10 to 700 Pa.

5. The hydrogel according to any one of the preceding claims, wherein the hydrogel has an elasticity module of from 35 to 480 Pa.

6. The hydrogel according to any one of the preceding claims, wherein the acrylamide and methylene bis-acrylamide are combined in a molar ratio of 150:1 to 1000:1.

7. The hydrogel according to claim 6, wherein the acrylamide and methylene bis-acrylamide are combined in a molar ratio of 200:1 to 600:1.

8. The hydrogel according to any one of the preceding claims comprising less than 15% by weight polyacrylamide.

9. The hydrogel according to claim 8 comprising less than 7.5% by weight polyacrylamide.

10. The hydrogel according to claim 9 comprising less than 3.5% by weight polyacrylamide.
